(19) Europäisches Patentamt
European Patent Office
Office européen des brevets

(11) EP 2 099 483 B1

(12) # FASCICULE DE BREVET EUROPEEN

(45) Date de publication et mention de la délivrance du brevet:
**25.12.2013 Bulletin 2013/52**

(21) Numéro de dépôt: **07866551.0**

(22) Date de dépôt: **30.11.2007**

(51) Int Cl.:
***A61K 39/12*** $^{(2006.01)}$ ***A61P 31/14*** $^{(2006.01)}$

(86) Numéro de dépôt international:
**PCT/FR2007/052431**

(87) Numéro de publication internationale:
**WO 2008/065315 (05.06.2008 Gazette 2008/23)**

(54) **METHODE D'IMMUNISATION CONTRE LES 4 SEROTYPES DE LA DENGUE**

IMMUNISIERUNGSPROTOKOLL GEGEN DIE VIER SEROTYPEN DES DENGUE-VIRUS

IMMUNIZATION PROTOCOL AGAINST THE 4 DENGUE SEROTYPES

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **01.12.2006 FR 0655255**

(43) Date de publication de la demande:
**16.09.2009 Bulletin 2009/38**

(73) Titulaire: **Sanofi Pasteur**
**69367 Lyon Cedex 07 (FR)**

(72) Inventeurs:
- **GUY, Bruno**
  **69005 Lyon (FR)**
- **BARBAN, Véronique**
  **69290 Craponne (FR)**
- **FORRAT, Rémi**
  **69780 Serezin Du Rhône (FR)**
- **LANG, Jean**
  **69780 Mions (FR)**

(74) Mandataire: **Commander, Paul Martin Brial**
**Mathys & Squire LLP**
**120 Holborn**
**London EC1N 2SQ (GB)**

(56) Documents cités:

| | |
|---|---|
| **EP-A- 1 159 968** | **WO-A-03/101397** |
| **WO-A1-2006/134433** | **WO-A1-2006/134443** |
| **WO-A1-2007/093472** | **WO-A1-2007/141259** |
| **WO-A1-2008/007021** | **WO-A2-01/60847** |
| **WO-A2-2008/047023** | |

- **GUIRAKHOO F ET AL: "CONSTRUCTION, SAFETY,AND IMMUNOGENICITY IN NONHUMAN PRIMATES OF A CHIMERIC YELLOW FEVER-DENGUE VIRUS TETRAVALENT VACCINE" JOURNAL OF VIROLOGY, THE AMERICAN SOCIETY FOR MICROBIOLOGY, US, vol. 75, no. 10, août 2001 (2001-08), pages 7290-7304, XP002961663 ISSN: 0022-538X**
- **BLANEY J E ET AL: "DEVELOPMENT OF A LIVE ATTENUATED DENGUE VIRUS VACCINE USING REVERSE GENETICS" VIRAL IMMUNOLOGY, MARY ANN LIEBERT, INC., NEW YORK, US, vol. 19, no. 1, avril 2006 (2006-04), pages 10-32, XP008068928 ISSN: 0882-8245**
- **SABCHAREON ARUNEE ET AL: "Safety and immunogenicity of tetravalent live-attenuated dengue vaccines in Thai adult volunteers: Role of serotype concentration, ratio, and multiple doses." AMERICAN JOURNAL OF TROPICAL MEDICINE AND HYGIENE, vol. 66, no. 3, mars 2002 (2002-03), pages 264-272, XP002441733 ISSN: 0002-9637**
- **GUIRAKHOO F ET AL: "Viremia and Immunogenicity in Nonhuman Primates of a Tetravalent Yellow Fever-Dengue Chimeric Vaccine: Genetic Reconstructions, Dose Adjustment, and Antibody Responses against Wild-type Dengue Virus Isolates" VIROLOGY, ACADEMIC PRESS,ORLANDO, US, vol. 298, no. 1, 20 juin 2002 (2002-06-20), pages 146-159, XP004469478 ISSN: 0042-6822**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 099 483 B1

- **HUANG C Y-H ET AL: "Chimeric dengue type 2 (vaccine strain PDK-53)/dengue type 1 virus as a potential candidate dengue type 1 virus vaccine", JOURNAL OF VIROLOGY, THE AMERICAN SOCIETY FOR MICROBIOLOGY, US, vol. 74, no. 7, 1 April 2000 (2000-04-01), pages 3020-3028, XP002174039, ISSN: 0022-538X, DOI: 10.1128/JVI.74.7.3020-3028.2000**

SEQUENCE LISTING

<110> sanofi Pasteur

<120> Méthode d'immunisation contre les quatre sérotypes de la Dengue

<130> PM 0608

<160> 2

<170> PatentIn version 3.3

<210> 1
<211> 10735
<212> DNA
<213> Dengue virus

<400> 1

```
agttgttagt ctacgtggac cgacaagaac agtttcgaat cggaagcttg cttaacgtag     60
ttctaacagt tttttattag agagcagatc tctgatgatc aaccaacgaa aaaagacggg    120
tcgaccgtct ttcaatatgc tgaaacgcgc gagaaaccgc gtgtcaactg tttcacagtt    180
ggcgaagaga ttctcaaaag gattgctctc aggccaagga cccatgaaat tggtgatggc    240
tttcatagca ttcttaagat ttctagccat acccccaaca gcaggaattt tggctagatg    300
gggctcattc aagaagaatg gagcgattaa agtgttacgg ggtttcaaga gagaaatctc    360
aaacatgcta aacataatga acaggaggaa aagatccgtg accatgctcc ttatgctgct    420
gcccacagcc ctggcgttcc atctgacgac acgaggggga gagccgcata tgatagttag    480
caagcaggaa agaggaaagt cacttttgtt caagacctct gcaggtgtca acatgtgcac    540
cctcattgcg atggatttgg gagagttgtg tgaggacacg atgacctaca aatgcccccg    600
gatcactgag gcggaaccag atgacgttga ctgttggtgc aatgccacgg acacatgggt    660
gacctatgga acgtgctctc aaactggcga acaccgacga gacaaacgtt ccgtcgcatt    720
ggccccacac gtggggcttg gcctagaaac aagagccgaa acgtggatgt cctctgaagg    780
tgcttggaaa cagatacaaa aagtagagac ttgggctctg agacatccag gattcacggt    840
gatagccctt tttctagcac atgccatagg aacatccatc acccagaaag ggatcatttt    900
cattttgctg atgctggtaa caccatctat ggccatgcga tgcgtgggaa taggcaacag    960
agacttcgtg gaaggactgt caggagcaac atgggtggat gtggtactgg agcatggaag   1020
ttgcgtcacc accatggcaa aaaacaaacc aacactggac attgaactct tgaagacgga   1080
ggtcacaaac cctgcagttc tgcgtaaatt gtgcattgaa gctaaaatat caaacaccac   1140
caccgattcg agatgtccaa cacaaggaga agccacactg gtggaagaac aagacgcgaa   1200
ctttgtgtgc cgacgaacgt tcgtggacag aggctggggc aatggctgtg ggctattcgg   1260
aaaaggtagt ctaataacgt gtgccaagtt taagtgtgtg acaaaactag aaggaaagat   1320
agctcaatat gaaaacctaa aatattcagt gatagtcacc gtccacactg gagatcagca   1380
ccaggtggga aatgagacta cagaacatgg aacaactgca accataacac ctcaagctcc   1440
tacgtcggaa atacagctga ccgactacgg aacccttaca ttagattgtt cacctaggac   1500
```

agggctagat tttaacgaga tggtgttgct gacaatgaaa aagaaatcat ggcttgtcca 1560
caaacagtgg tttctagact taccactgcc ttggacctct ggggctttaa catcccaaga 1620
gacttggaac agacaagatt tactggtcac atttaagaca gctcatgcaa agaagcagga 1680
agtagtcgta ctaggatcac aagaaggagc aatgcacact gcgctgactg gagcgacaga 1740
aatccaaacg tcaggaacga caacaatttt cgcaggacac ctaaaatgca gactaaaaat 1800
ggacaaacta actttaaaag ggatgtcata tgtgatgtgc acaggctcat tcaagttaga 1860
gaaagaagtg gctgagaccc agcatggaac tgttctggtg caggttaaat atgaaggaac 1920
agacgcacca tgcaagattc ccttttcgac ccaagatgag aaaggagcaa cccagaatgg 1980
gagattaata acagccaacc ccatagtcac tgacaaagaa aaaccagtca atattgaggc 2040
agaaccaccc tttggtgaga gctacatcgt ggtaggagca ggtgaaaaag ctttgaaact 2100
aagctggttc aagaaaggaa gcagcatagg gaaaatgttt gaagcaactg cccgaggagc 2160
acgaaggatg gccattctgg gagacaccgc atgggacttc ggttctatag gaggagtgtt 2220
cacgtctatg ggaaaactgg tacaccaggt tttggaact gcatatggag ttttgtttag 2280
cggagtttct tggaccatga aaataggaat agggattctg ctgacatggc taggattaaa 2340
ttcaaggaac acgtcccttt cggtgatgtg catcgcagtt ggcatggtca cactgtacct 2400
aggagtcatg gttcaggcag attcgggatg tgtaatcaac tggaaaggca gagaacttaa 2460
atgtggaagc ggcatttttg tcactaatga agttcacact tggacagagc aatacaaatt 2520
ccaggctgac tcccccaaga gactatcagc agccattggg aaggcatggg aggagggtgt 2580
gtgtggaatc cgatcagcca ctcgtctcga gaacatcatg tggaaacaaa tatcaaatga 2640
attgaaccac atcctacttg aaaatgacat gaaatttaca gtggtcgtgg gagacgttag 2700
tggaatcttg gcccaaggaa aaaaaatgat taggccacaa cccatggaac acaaatactc 2760
gtggaaaagc tggggaaaag ctaaaatcat aggagcggat gtacagaaca ccaccttcat 2820
catcgacggc ccaaacaccc cagaatgccc tgacaatcaa agagcatgga atatttggga 2880
agtagaggac tatggatttg ggattttcac gacaaacata tggttgaaat tgcgtgactc 2940
ctacacccaa gtatgtgacc accggctgat gtcagctgcc attaaggaca gcaaggcagt 3000
ccatgctgac atggggtact ggatagaaag tgaaaagaac gagacatgga agttggcgag 3060
agcctccttt atagaagtta agacatgcat ctggccaaaa tcccacactc tatggagcaa 3120
tggagttctg gaaagtgaaa tgataattcc aaagatatat ggaggaccaa tatctcagca 3180
caactacaga ccaggatatt tcacacaaac agcagggccg tggcacctag gcaagttgga 3240
actagatttc gatttttgtg aaggtaccac agttgttgtg gatgaacatt gtggaaatcg 3300
aggaccatct ctcagaacca caacagtcac aggaaagata atccatgaat ggtgctgcag 3360
atcttgtacg ctacccccc tacgtttcaa aggggaagac gggtgttggt acggcatgga 3420
aatcagacca gtgaaggaca aggaagagaa cctggtcaag tcaatggtct ctgcagggtc 3480
aggagaagtg gacagctttt cactaggact gctatgcata tcaataatga ttgaagaagt 3540

gatgagatcc agatggagca aaaaaatgct gatgactgga acactggctg tgttcctcct 3600
tcttataatg ggacaattga catggagtga tctgatcagg ttatgtatta tggttggagc 3660
caacgcttca gacaagatgg ggatgggaac aacgtaccta gctttaatgg ccactttcaa 3720
aatgagacca atgttcgccg tcgggctatt atttcgcaga ctaacatcta gagaagttct 3780
tcttcttaca attggcttga gcctggtggc atccgtggag ctaccaagtt ccctagagga 3840
gctgggggat ggacttgcaa taggcatcat gatgttgaaa ttattgactg attttcagtc 3900
acaccagcta tgggctactc tgctatcctt gacatttatt aaaacaactt tttcattgca 3960
ctatgcatgg aagacaatgg ctatggtact gtcaattgta tctctcttcc ctttatgcct 4020
gtccacgacc tctcaaaaaa caacatggct tccggtgctg ttgggatctc ttggatgcaa 4080
accactaccc atgtttctta taacagaaaa caaaatctgg ggaaggaaga gttggcccct 4140
caatgaagga attatggctg ttggaatagt tagtattcta ctaagttcac ttttaaaaaa 4200
tgatgtgccg ctagccggcc cattaatagc tggaggcatg ctaatagcat gttatgtcat 4260
atccggaagc tcagctgatt tatcactgga gaaagcggct gaggtctcct gggaggaaga 4320
agcagaacac tcaggcgcct cacacaacat actagtagag gttcaagatg atggaaccat 4380
gaagataaaa gatgaagaga gagatgacac gctcaccatt ctccttaaag caactctgct 4440
ggcagtctca ggggtgtacc caatgtcaat accagcgacc ctttttgtgt ggtatttttg 4500
gcagaaaaag aaacagagat caggagtgct atgggacaca cccagccccc cagaagtgga 4560
aagagcagtt cttgatgatg gcatctatag aattttgcaa agaggactgt tgggcaggtc 4620
ccaagtagga gtaggagttt tccaagaagg cgtgttccac acaatgtggc acgtcactag 4680
gggagctgtc ctcatgtatc aaggaaaaag gctggaacca agctgggcca gtgtcaaaaa 4740
agacttgatc tcatatggag gaggttggag gtttcaagga tcctggaaca cgggagaaga 4800
agtacaggtg attgctgttg aaccgggaaa aaaccccaaa aatgtacaaa caacgccggg 4860
taccttcaag acccctgaag gcgaagttgg agccatagcc ttagacttta aacctggcac 4920
atctggatct cccatcgtaa acagagaggg aaaaatagta ggtctttatg gaaatggagt 4980
ggtgacaaca agcggaactt acgttagtgc catagctcaa gctaaggcat cacaagaagg 5040
gcctctacca gagattgagg acaaggtgtt taggaaaaga aacttaacaa taatggacct 5100
acatccagga tcgggaaaaa caagaagata ccttccagcc atagtccgtg aggccataaa 5160
aaggaagctg cgcacgctaa tcctagctcc cacaagagtt gtcgcttctg aaatggcaga 5220
ggcactcaag ggagtgccaa taaggtatca gacaacagca gtgaagagtg aacacacagg 5280
aaaggagata gttgacctta tgtgccacgc cactttcacc atgcgcctcc tgtctcccgt 5340
gagagttccc aattataaca tgattatcat ggatgaagca cacttcaccg atccagccag 5400
catagcagcc agagggtaca tctcaacccg agtgggtatg ggtgaagcag ctgcgatctt 5460
tatgacagcc actcccccag gatcggtgga ggcctttcca cagagcaatg caattatcca 5520
agatgaggaa agagacattc ctgagagatc atggaactca ggctatgact ggatcactga 5580

ttttccaggt aaaacagtct ggtttgttcc aagcatcaaa tcaggaaatg acattgccaa 5640
ctgtttaaga aaaaacggga aacgggtgat ccaattgagc agaaaaacct ttgacactga 5700
gtaccagaaa acaaaaaaca acgactggga ctatgtcgtc acaacagaca tttccgaaat 5760
gggagcaaat ttccgggccg acagggtaat agacccaagg cggtgtctga aaccggtaat 5820
actaaaagat ggtccagagc gcgtcattct agccggaccg atgccagtga ctgtggccag 5880
tgccgcccag aggagaggaa gaattggaag gaaccaaaac aaggaaggtg atcagtatat 5940
ttacatggga cagcctttaa aaaatgatga ggaccacgct cattggacag aagcaaagat 6000
gctccttgac aatataaaca caccagaagg gattatccca gccctctttg agccggagag 6060
agaaaagagt gcagctatag acggggaata cagactgcgg ggtgaagcaa ggaaaacgtt 6120
cgtggagctc atgagaagag gggatctacc agtctggcta tcctacaaag ttgcctcaga 6180
aggcttccag tactccgaca gaaggtggtg cttcgatggg gaaaggaaca accaggtgtt 6240
ggaggagaac atggacgtgg agatctggac aaaagaagga gaaagaaaga aactacgacc 6300
tcgctggttg gacgccagaa catactctga cccactggct ctgcgcgagt ttaaagagtt 6360
tgcagcagga agaagaagcg tctcaggtga cctaatatta gaaataggga aacttccaca 6420
acatttgacg caaagggccc agaatgcttt ggacaacttg gtcatgttgc acaattccga 6480
acaaggagga aaagcctata gacatgctat ggaagaactg ccagacacaa tagaaacgtt 6540
gatgctccta gccttgatag ctgtgttgac tggtggagtg acgctgttct tcctatcagg 6600
aagaggtcta ggaaaaacat ctatcggctt actctgcgtg atggcctcaa gcgcactgtt 6660
atggatggcc agtgtggagc cccattggat agcggcctcc atcatactgg agttctttct 6720
gatggtactg cttattccag agccagacag acagcgcact ccacaggaca accagctagc 6780
atatgtggtg ataggtctgt tattcgtgat attgacagtg gcagccaatg agatgggatt 6840
attggaaacc acaaagaaag acctggggat tggccatgta gctgctgaaa accaccacca 6900
tgctacaatg ctggacgtag acctacatcc agcttcagcc tggaccctct atgcagtggc 6960
cacaacaatc atcactccta tgatgagaca cacaattgaa aacacaacgg caaatatttc 7020
cctgacagcc atcgcaaacc aagcagctat attgatggga cttgacaagg gatggccaat 7080
atcgaagatg gacataggag ttccacttct cgccttgggg tgctattccc aagtgaatcc 7140
gctgacactg atagcggcag tattgatgct agtagctcat tacgccataa ttggacctgg 7200
actgcaagca aaagctacta gagaagctca aaaaagaaca gcggctggaa taatgaaaaa 7260
tccaactgtc gacgggattg ttgcaataga cttagatccc gtggtttacg atgcaaaatt 7320
tgaaaaacag ctaggccaaa taatgttgtt gatactttgc acatcacaga ttcttttgat 7380
gcggactaca tgggccttgt gtgaatccat cacattggct actggacctc tgaccactct 7440
ttgggaggga tctccaggaa aattctggaa caccacaata gcggtatcca tggcaaacat 7500
tttcaggggg agttatctag caggagcagg tctggccttc tcattaatga aatctctagg 7560
aggaggtagg agaggcacgg gagcccaagg ggaaacactg ggagaaaaat ggaaaagaca 7620

actaaaccaa ctgagcaagt cagaattcaa tacttacaag aggagtggga ttatggaggt 7680
ggatagatcc gaagccaaag agggactgaa aagaggagaa acaaccaaac acgcagtatc 7740
gagaggaacg gccaaactga ggtggttcgt ggagaggaac cttgtgaaac cagaagggaa 7800
agtcatagac ctcggttgtg gaagaggtgg ctggtcatat tattgcgctg ggctgaagaa 7860
agtcacagaa gtgaaaggat acacaaaagg aggacctgga catgaggaac caatcccaat 7920
ggcgacctat ggatggaacc tagtaaggct gcactccgga aaagatgtat tttttatacc 7980
acctgagaaa tgtgacaccc ttttgtgtga tattggtgag tcctctccga acccaactat 8040
agaggaagga agaacgttac gtgttctgaa aatggtggaa ccatggctca gaggaaacca 8100
attttgcata aaaattctaa atccctatat gccgagcgtg gtagaaactc tggaacaaat 8160
gcaaagaaaa catggaggaa tgctagtgcg aaacccactc tcaagaaatt ccacccatga 8220
aatgtactgg gtttcatgtg gaacaggaaa cattgtgtca gcagtaaaca tgacatctag 8280
aatgttgcta aatcggttca caatggctca caggaagcca acatatgaaa gagacgtgga 8340
cttaggcgct ggaacaagac atgtggcagt agaaccagag gtagccaacc tagatatcat 8400
tggccagagg atagagaata taaaaaatga acataagtca acatggcatt atgatgagga 8460
caatccatac aaaacatggg cctatcatgg atcatatgag gttaagccat caggatcggc 8520
ctcatccatg gtcaatggcg tggtgagatt gctcaccaaa ccatgggatg ttatccccat 8580
ggtcacacaa atagccatga ctgataccac accctttgga caacagaggg tgtttaaaga 8640
gaaagttgac acgcgcacac caaaagcaaa acgtggcaca gcacaaatta tggaagtgac 8700
agccaggtgg ttatggggtt tcctttctag aaacaaaaaa cccagaattt gcacaagaga 8760
ggagtttaca agaaaagtta ggtcaaacgc agctattgga gcagtgttcg ttgatgaaaa 8820
tcaatggaac tcggcaaaag aagcagtgga agacgaacgg ttctgggaac ttgtccacag 8880
agagagggag cttcataaac aggggaaatg tgccacgtgt gtctacaata tgatggggaa 8940
gagagagaaa aaattaggag agttcggaaa ggcaaaagga agtcgtgcaa tatggtacat 9000
gtggttggga gcacgcttcc tagagtttga agcccttggt ttcatgaatg aagatcactg 9060
gttcagtaga gagaattcac tcagtggagt ggaaggagaa ggactccaca aacttggata 9120
catactcaga gacatatcaa ggattccagg ggggaacatg tatgcagatg acacagccgg 9180
atgggacaca agaataacag aggatgatct ccagaatgag gctaaaatca ctgacatcat 9240
ggagcccgaa catgccctgc tggctacgtc aatctttaag ctgacctacc aaaataaggt 9300
ggtaagggtg cagagaccag caaaaaatgg aaccgtgatg gatgttatat ccagacgtga 9360
ccagagaggc agtggacagg ttggaactta tggcttaaac actttcacca acatggaggc 9420
ccaactgata agacaaatgg agtctgaggg aatcttttta cccagcgaat tggaaacccc 9480
aaatctagcc ggaagagttc tcgactggtt ggaaaaatat ggtgtcgaaa ggctgaaaag 9540
aatggcaatc agcggagatg actgtgtggt gaaaccaatt gatgacaggt tcgcaacagc 9600
cttaacagct ttgaatgaca tgggaaaagt aagaaaagac ataccacaat gggaaccttc 9660

aaaaggatgg aatgattggc aacaagtgcc tttctgttca caccacttcc accagctaat 9720
tatgaaggat gggagggaga tagtggtgcc atgccgcaac caagatgaac ttgtggggag 9780
ggccagagta tcacaaggcg ccggatggag cctgagagaa accgcatgcc taggcaagtc 9840
atatgcacaa atgtggcagc tgatgtattt ccacaggaga gacctgagac tggcggctaa 9900
cgctatttgt tcagccgttc cagttgattg ggtcccaacc agccgcacca cctggtcgat 9960
ccatgcccat caccaatgga tgacaacaga agacatgtta tcagtatgga atagggtctg 10020
gatagaggaa aacccatgga tggaggataa gactcatgtg tccagttggg aagaagttcc 10080
atacctagga aagagggaag atcagtggtg tggatccctg ataggcttaa cagcaagggc 10140
cacctgggcc actaatatac aagtggccat aaaccaagtg agaaggctca ttgggaatga 10200
gaattatcta gattacatga catcaatgaa gagattcaag aatgagagtg atcccgaagg 10260
ggcactctgg taagtcaaca cattcacaaa ataaaggaaa ataaaaaatc aaatgaggca 10320
agaagtcagg ccagattaag ccatagtacg gtaagagcta tgctgcctgt gagccccgtc 10380
caaggacgta aaatgaagtc aggccgaaag ccacggtttg agcaagccgt gctgcctgtg 10440
gctccatcgt ggggatgtaa aaacccggga ggctgcaacc catggaagct gtacgcatgg 10500
ggtagcagac tagtggttag aggagacccc tcccaagaca caacgcagca gcggggccca 10560
acaccagggg aagctgtacc ctggtggtaa ggactagagg ttagaggaga ccccccgcgt 10620
aacaataaac agcatattga cgctgggaga gaccagagat cctgctgtct ctacagcatc 10680
attccaggca cagaacgcca gaaaatggaa tggtgctgtt gaatcaacag gttct 10735

<210> 2
<211> 10723
<212> DNA
<213> Dengue virus

<400> 2

```
agttgttagt ctacgtggac cgacaaagac agattctttg agggagctaa gctcaatgta    60
gttctaacag tttttaatt agagagcaga tctctgatga ataaccaacg gaaaaaggcg    120
aaaaacacgc ctttcaatat gctgaaacgc gagagaaacc gcgtgtcgac tgtgcaacag    180
ctgacaaaga gattctcact tggaatgctg cagggacgag gaccattaaa actgttcatg    240
gccctggtgg cgttccttcg tttcctaaca atcccaccaa cagcagggat attgaagaga    300
tggggaacaa ttaaaaaatc aaaagctatt aatgttttga gagggttcag gaaagagatt    360
ggaaggatgc tgaacatctt gaataggaga cgcagatctg caggcatgat cattatgctg    420
attccaacag tgatggcgtt ccatttaacc acacgtaacg gagaaccaca catgatcgtc    480
agcagacaag agaaagggaa aagtcttctg tttaaaacag aggttggcgt gaacatgtgt    540
accctcatgg ccatggacct tggtgaattg tgtgaagaca caatcacgta caagtgtccc    600
cttctcaggc agaatgagcc agaagacata gactgttggt gcaactctac gtccacgtgg    660
gtaacttatg ggacgtgtac caccatggga gaacatagaa gagaaaaaag atcagtggca    720
ctcgttccac atgtgcgaat gggactggag acacgaactg aaacatggat gtcatcagaa    780
```

gggcctgga aacatgtcca gagaattgaa acttggatct tgagacatcc aggcttcacc 840
atgatggcag caatcctggc atacaccata ggaacgacac atttccaaag agccctgatt 900
ttcatcttac tgacagctgt cactccttca atgacaatgc gttgcatagg aatgtcaaat 960
agagactttg tggaaggggt ttcaggagga agctgggttg acatagtctt agaacatgga 1020
agctgtgtga cgacgatggc aaaaaacaaa ccaacattgg attttgaact gataaaaaca 1080
gaagccaaac agcctgccac cctaaggaag tactgtatag aggcaaagct aaccaacaca 1140
acaacagaat ctcgctgccc aacacaaggg gaacccagcc taaatgaaga gcaggacaaa 1200
aggttcgtct gcaaacactc catggtagac agaggatggg gaaatggatg tggactattt 1260
ggaaagggag gcattgtgac ctgtgctatg ttcagatgca aaaagaacat ggaaggaaaa 1320
gttgtgcaac cagaaaactt ggaatacacc attgtgataa cacctcactc aggggaagag 1380
catgcagtcg gaaatgacac aggaaaacat ggcaaggaaa tcaaaataac accacagagt 1440
tccatcacag aagcagaatt gacaggttat ggcactgtca caatggagtg ctctccaaga 1500
acgggcctcg acttcaatga gatggtgttg ctgcagatgg aaaataaagc ttggctggtg 1560
cacaggcaat ggttcctaga cctgccgtta ccatggttgc ccggagcgga cacacaagag 1620
tcaaattgga tacagaagga gacattggtc actttcaaaa atccccatgc gaagaaacag 1680
gatgttgttg ttttaggatc ccaagaaggg gccatgcaca cagcacttac aggggccaca 1740
gaaatccaaa tgtcatcagg aaacttactc ttcacaggac atctcaagtg caggctgaga 1800
atggacaagc tacagctcaa aggaatgtca tactctatgt gcacaggaaa gtttaaagtt 1860
gtgaaggaaa tagcagaaac acaacatgga acaatagtta tcagagtgca atatgaaggg 1920
gacggctctc catgcaagat cccttttgag ataatggatt tggaaaaaag acatgtctta 1980
ggtcgcctga ttacagtcaa cccaattgtg acagaaaaag atagcccagt caacatagaa 2040
gcagaacctc catttggaga cagctacatc atcataggag tagagccggg acaactgaag 2100
ctcaactggt ttaagaaagg aagttctatc ggccaaatgt ttgagacaac aatgaggggg 2160
gcgaagagaa tggccatttt aggtgacaca gcctgggatt ttggatcctt gggaggagtg 2220
tttacatcta taggaaaggc tctccaccaa gtctttggag caatctatgg agctgccttc 2280
agtggggttt catggactat gaaaatcctc ataggagtca ttatcacatg gataggaatg 2340
aattcacgca gcacctcact gtctgtgaca ctagtattgg tgggaattgt gacactgtat 2400
ttgggagtca tggtgcaggc cgatagtggt tgcgttgtga gctggaaaaa caaagaactg 2460
aaatgtggca gtgggatttt catcacagac aacgtgcaca catggacaga acaatacaaa 2520
ttccaaccag aatccccttc aaaactagct tcagctatcc agaaagccca tgaagaggac 2580
atttgtggaa tccgctcagt aacaagactg gagaatctga tgtggaaaca aataacacca 2640
gaattgaatc acattctatc agaaaatgag gtgaagttaa ctattatgac aggagacatc 2700
aaaggaatca tgcaggcagg aaaacgatct ctgcggcctc agcccactga gctgaagtat 2760
tcatggaaaa catggggcaa agcaaaaatg ctctctacag agtctcataa ccagaccttt 2820

ctcattgatg gccccgaaac agcagaatgc cccaacacaa atagagcttg gaattcgttg 2880
gaagttgaag actatggctt tggagtattc accaccaata tatggctaaa attgaaagaa 2940
aaacaggatg tattctgcga ctcaaaactc atgtcagcgg ccataaaaga caacagagcc 3000
gtccatgccg atatgggtta ttggatagaa agtgcactca atgacacatg gaagatagag 3060
aaagcctctt tcattgaagt taaaaactgc cactggccaa aatcacacac cctctggagc 3120
aatggagtgc tagaaagtga gatgataatt ccaaagaatc tcgctggacc agtgtctcaa 3180
cacaactata gaccaggcta ccatacacaa ataacaggac catggcatct aggtaagctt 3240
gagatggact ttgatttctg tgatggaaca acagtggtag tgactgagga ctgcggaaat 3300
agaggaccct ctttgagaac aaccactgcc tctggaaaac tcataacaga atggtgctgc 3360
cgatcttgca cattaccacc gctaagatac agaggtgagg atgggtgctg gtacgggatg 3420
gaaatcagac cattgaagga gaaagaagag aatttggtca actccttggt cacagctgga 3480
catgggcagg tcgacaactt ttcactagga gtcttgggaa tggcattgtt cctggaggaa 3540
atgcttagga cccgagtagg aacgaaacat gcaatactac tagttgcagt ttcttttgtg 3600
acattgatca cagggaacat gtcctttaga gacctgggaa gagtgatggt tatggtaggc 3660
gccactatga cggatgacat aggtatgggc gtgacttatc ttgccctact agcagccttc 3720
aaagtcagac caacttttgc agctggacta ctcttgagaa agctgacctc caaggaattg 3780
atgatgacta ctataggaat tgtactcctc tcccagagca ccataccaga gaccattctt 3840
gagttgactg atgcgttagc cttaggcatg atggtcctca aaatggtgag aaatatggaa 3900
aagtatcaat tggcagtgac tatcatggct atcttgtgcg tcccaaacgc agtgatatta 3960
caaaacgcat ggaaagtgag ttgcacaata ttggcagtgg tgtccgtttc cccactgttc 4020
ttaacatcct cacagcaaaa aacagattgg ataccattag cattgacgat caaaggtctc 4080
aatccaacag ctatttttct aacaaccctc tcaagaacca gcaagaaaag gagctggcca 4140
ttaaatgagg ctatcatggc agtcgggatg gtgagcattt tagccagttc tctcctaaaa 4200
aatgatattc ccatgacagg accattagtg gctggagggc tcctcactgt gtgctacgtg 4260
ctcactggac gatcggccga tttggaactg gagagagcag ccgatgtcaa atgggaagac 4320
caggcagaga tatcaggaag cagtccaatc ctgtcaataa caatatcaga agatggtagc 4380
atgtcgataa aaaatgaaga ggaagaacaa acactgacca tactcattag aacaggattg 4440
ctggtgatct caggactttt tcctgtatca ataccaatca cggcagcagc atggtacctg 4500
tgggaagtga agaaacaacg ggccggagta ttgtgggatg ttccttcacc cccacccatg 4560
ggaaaggctg aactggaaga tggagcctat agaattaagc aaaaagggat tcttggatat 4620
tcccagatcg gagccggagt ttacaaagaa ggaacattcc atacaatgtg gcatgtcaca 4680
cgtggcgctg ttctaatgca taaaggaaag aggattgaac caacatgggc ggacgtcaag 4740
aaagacctaa tatcatatgg aggaggctgg aagttagaag gagaatggaa ggaaggagaa 4800
gaagtccagg tattggcact ggagcctgga aaaaatccaa gagccgtcca aacgaaacct 4860

ggtcttttca aaaccaacgc cggaacaata ggtgctgtat ctctggactt ttctcctgga 4920
acgtcaggat ctccaattat cgacaaaaaa ggaaaagttg tgggtcttta tggtaatggt 4980
gttgttacaa ggagtggagc atatgtgagt gctatagccc agactgaaaa aagcattgaa 5040
gacaacccag agatcgaaga tcacattttc cgaaagagaa gactgaccat catggacctc 5100
cacccaggag cgggaaagac gaagagatac cttccggcca tagtcagaga agctataaaa 5160
cggggtttga gaacattaat cttggccccc actagagttg tggcagctga aatggaggaa 5220
gcccttagag gacttccaat aagataccag accccagcca tcagagctga gcacaccggg 5280
cgggagattg tggacctaat gtgtcatgcc acatttacca tgaggctgct atcaccagtt 5340
agagtgccaa actacaacct gattatcatg gacgaagccc atttcacaga cccagcaagt 5400
atagcagcta gaggatacat ctcaactcga gtggagatgg gtgaggcagc tgggattttt 5460
atgacagcca ctcccccggg aagcagagac ccatttcctc agagcaatgc accaatcata 5520
gatgaagaaa gagaaatccc tgaacgctcg tggaattccg gacatgaatg ggtcacggat 5580
tttaaaggga agactgtttg gttcgttcca agtataaaag caggaaatga tatagcagct 5640
tgcctgagga aaaatggaaa gaaagtgata caactcagta ggaagacctt tgattctgag 5700
tatgtcaaga ctagaaccaa tgattgggac ttcgtggtta caactgacat ttcagaaatg 5760
ggtgccaatt tcaaggctga gagggttata gaccccagac gctgcatgaa accagtcata 5820
ctaacagatg gtgaagagcg ggtgattctg gcaggaccta tgccagtgac ccactctagt 5880
gcagcacaaa gaagagggag aataggaaga aatccaaaaa atgagaatga ccagtacata 5940
tacatggggg aacctctgga aaatgatgaa gactgtgcac actggaaaga agctaaaatg 6000
ctcctagata acatcaacac gccagaagga atcattccta gcatgttcga accagagcgt 6060
gaaaaggtgg atgccattga tggcgaatac cgcttgagag gagaagcaag gaaaaccttt 6120
gtagacttaa tgagaagagg agacctacca gtctggttgg cctacagagt ggcagctgaa 6180
ggcatcaact acgcagacag aaggtggtgt tttgatggag tcaagaacaa ccaaatccta 6240
gaagaaaacg tggaagttga aatctggaca aaagaagggg aaaggaagaa attgaaaccc 6300
agatggttgg atgctaggat ctattctgac ccactggcgc taaaagaatt taaggaattt 6360
gcagccggaa gaaagtctct gaccctgaac ctaatcacag aaatgggtag gctcccaacc 6420
ttcatgactc agaaggcaag agacgcactg gacaacttag cagtgctgca cacggctgag 6480
gcaggtggaa gggcgtacaa ccatgctctc agtgaactgc cggagaccct ggagacattg 6540
cttttactga cacttctggc tacagtcacg ggagggatct tttattctt gatgagcgca 6600
aggggcatag ggaagatgac cctgggaatg tgctgcataa tcacggctag catcctccta 6660
tggtacgcac aaatacagcc acactggata gcagcttcaa taatactgga gttttttctc 6720
atagttttgc ttattccaga acctgaaaaa cagagaacac cccaagacaa ccaactgacc 6780
tacgttgtca tagccatcct cacagtggtg gccgcaacca tggcaaacga gatgggtttc 6840
ctagaaaaaa cgaagaaaga tctcggattg ggaagcattg caacccagca acccgagagc 6900

aacatcctgg acatagatct acgtcctgca tcagcatgga cgctgtatgc cgtggccaca 6960
acatttgtta caccaatgtt gagacatagc attgaaaatt cctcagtgaa tgtgtcccta 7020
acagctatag ccaaccaagc cacagtgtta atgggtctcg ggaaaggatg gccattgtca 7080
aagatggaca tcggagttcc ccttctcgcc attggatgct actcacaagt caaccccata 7140
actctcacag cagctctttt cttattggta gcacattatg ccatcatagg gccaggactc 7200
caagcaaaag caaccagaga agctcagaaa agagcagcgg cgggcatcat gaaaaaccca 7260
actgtcgatg gaataacagt gattgaccta gatccaatac cttatgatcc aaagtttgaa 7320
aagcagttgg gacaagtaat gctcctagtc ctctgcgtga ctcaagtatt gatgatgagg 7380
actacatggg ctctgtgtga ggctttaacc ttagctaccg ggcccatctc cacattgtgg 7440
gaaggaaatc cagggaggtt ttggaacact accattgcgg tgtcaatggc taacattttt 7500
agagggagtt acttggccgg agctggactt ctcttttcta ttatgaagaa cacaaccaac 7560
acaagaaggg gaactggcaa cataggagag acgcttggag agaaatggaa aagccgattg 7620
aacgcattgg gaaaaagtga attccagatc tacaagaaaa gtggaatcca ggaagtggat 7680
agaaccttag caaaagaagg cattaaaaga ggagaaacgg accatcacgc tgtgtcgcga 7740
ggctcagcaa aactgagatg gttcgttgag agaaacatgg tcacaccaga agggaaagta 7800
gtggacctcg gttgtggcag aggaggctgg tcatactatt gtggaggact aaagaatgta 7860
agagaagtca aaggcctaac aaaaggagga ccaggacacg aagaacccat ccccatgtca 7920
acatatgggt ggaatctagt gcgtcttcaa agtggagttg acgttttctt catcccgcca 7980
gaaaagtgtg acacattatt gtgtgacata ggggagtcat caccaaatcc cacagtggaa 8040
gcaggacgaa cactcagagt ccttaactta gtagaaaatt ggttgaacaa caacactcaa 8100
ttttgcataa aggttctcaa cccatatatg ccctcagtca tagaaaaaat ggaagcacta 8160
caaaggaaat atggaggagc cttagtgagg aatccactct cacgaaactc cacacatgag 8220
atgtactggg tatccaatgc ttccgggaac atagtgtcat cagtgaacat gatttcaagg 8280
atgttgatca acagatttac aatgagatac aagaaagcca cttacgagcc ggatgttgac 8340
ctcggaagcg gaacccgtaa catcgggatt gaaagtgaga taccaaacct agatataatt 8400
gggaaaagaa tagaaaaaat aaagcaagag catgaaacat catggcacta tgaccaagac 8460
cacccataca aaacgtgggc ataccatggt agctatgaaa caaaacagac tggatcagca 8520
tcatccatgg tcaacggagt ggtcaggctg ctgacaaaac cttgggacgt tgtccccatg 8580
gtgacacaga tggcaatgac agacacgact ccatttggac aacagcgcgt tttaaagag 8640
aaagtggaca cgagaaccca agaaccgaaa gaaggcacga agaaactaat gaaaataaca 8700
gcagagtggc tttggaaaga attagggaag aaaaagacac ccaggatgtg caccagagaa 8760
gaattcacaa gaaaggtgag aagcaatgca gccttggggg ccatattcac tgatgagaac 8820
aagtggaagt cggcacgtga ggctgttgaa gatagtaggt tttgggagct ggttgacaag 8880
gaaaggaatc tccatcttga aggaaagtgt gaaacatgtg tgtacaacat gatgggaaaa 8940

```
agagagaaga agctagggga attcggcaag gcaaaaggca gcagagccat atggtacatg    9000
tggcttggag cacgcttctt agagtttgaa gccctaggat tcttaaatga agatcactgg    9060
ttctccagag agaactccct gagtggagtg gaaggagaag ggctgcacaa gctaggttac    9120
attctaagag acgtgagcaa gaaagaggga ggagcaatgt atgccgatga caccgcagga    9180
tgggatacaa aaatcacact agaagaccta aaaaatgaag agatggtaac aaaccacatg    9240
gaaggagaac acaagaaact agccgaggcc attttcaaac taacgtacca aaacaaggtg    9300
gtgcgtgtgc aaagaccaac accaagaggc acagtaatgg acatcatatc gagaagagac    9360
caaagaggta gtggacaagt tggcacctat ggactcaata ctttcaccaa tatggaagcc    9420
caactaatca gacagatgga gggagaagga gtctttaaaa gcattcagca cctaacaatc    9480
acagaagaaa tcgctgtgca aaactggtta gcaagagtgg ggcgcgaaag gttatcaaga    9540
atggccatca gtggagatga ttgtgttgtg aaacctttag atgacaggtt cgcaagcgct    9600
ttaacagctc taaatgacat gggaaagatt aggaaagaca tacaacaatg ggaaccttca    9660
agaggatgga atgattggac acaagtgccc ttctgttcac accatttcca tgagttaatc    9720
atgaaagacg gtcgcgtact cgttgttcca tgtagaaacc aagatgaact gattggcaga    9780
gcccgaatct cccaaggagc agggtggtct ttgcgggaga cggcctgttt ggggaagtct    9840
tacgcccaaa tgtggagctt gatgtacttc cacagacgcg acctcaggct ggcggcaaat    9900
gctatttgct cggcagtacc atcacattgg gttccaacaa gtcgaacaac ctggtccata    9960
catgctaaac atgaatggat gacaacggaa gacatgctga cagtctggaa cagggtgtgg   10020
attcaagaaa acccatggat ggaagacaaa actccagtgg aaacatggga ggaaatccca   10080
tacttgggga aaagagaaga ccaatggtgc ggctcattga ttgggttaac aagcagggcc   10140
acctgggcaa agaacatcca agcagcaata aatcaagtta gatcccttat aggcaatgaa   10200
gaatacacag attacatgcc atccatgaaa agattcagaa gagaagagga agaagcagga   10260
gttctgtggt agaaagcaaa actaacatga aacaaggcta gaagtcaggt cggattaagc   10320
catagtacgg aaaaaactat gctacctgtg agccccgtcc aaggacgtta aaagaagtca   10380
ggccatcata aatgccatag cttgagtaaa ctatgcagcc tgtagctcca cctgagaagg   10440
tgtaaaaaat ccgggaggcc acaaaccatg gaagctgtac gcatggcgta gtggactagc   10500
ggttagggga gacccctccc ttacaaatcg cagcaacaat gggggcccaa ggcgagatga   10560
agctgtagtc tcgctggaag gactagaggt tagaggagac ccccccgaaa caaaaaacag   10620
catattgacg ctgggaaaga ccagagatcc tgctgtctcc tcagcatcat tccaggcaca   10680
gaacgccaga aaatggaatg gtgctgttga atcaacaggt tct                      10723
```

**Description**

La description concerne une méthode pour induire une protection contre les 4 sérotypes de la dengue chez un patient, comprenant :

(a) une première administration d'un vaccin monovalent comprenant un virus vaccinal de la dengue d'un premier sérotype
(b) une deuxième administration d'un vaccin tétravalent comprenant des virus vaccinaux des 4 serotypes de la dengue, et

dans laquelle la deuxième administration (b) est mise en oeuvre au moins 30 jours à au plus 12 mois après la première administration (a).

Les maladies dengue sont causées par quatre virus du genre flavivirus de type sérologique proches mais distincts d'un point de vue antigénique (Gübler et al., 1988 In: Epidemiology of arthropod-bome viral disease. Monath TPM, editor, Boca Raton (FL): CRC Press: 223-60 ; Kautner et al., 1997, J. of Pediatrics, 131:516-524; Rigau-Pérez et al., 1998, Lancet; 352: 971-977 ; Vaughn et al., 1997, J Infect Dis; 176: 322-30). L'infection avec un sérotype de la dengue peut produire un spectre de maladie clinique allant d'un syndrome viral non spécifique jusqu'à une maladie sévère hémorragique fatale. La période d'incubation de la fièvre dengue après piqûre du moustique est d'environ 4 jours (allant de 3 à 14 jours). La fièvre dengue est caractérisée par une fièvre biphasique, des maux de tête, des douleurs dans différentes parties du corps, une prostration, des éruptions, et une lymphadénopathie, (Kautner et al., 1997, J. of Pediatrics, 131:516-524 ; Rigau-Pérez et al., 1998, Lancet; 352: 971-977). La période virémique est la même que la période fébrile (Vaughn et al., 1997, J. Infect. Dis.; 176: 322-30). La guérison de la fièvre dengue est acquise au bout de 7 à 10 jours, mais une asthénie prolongée est usuelle. Des baisses de la numération des leucocytes et des plaquettes sont fréquentes.

La dengue hémorragique est une maladie fébrile sévère caractérisée par des anomalies de l'homéostasie et une augmentation de la perméabilité vasculaire qui peut conduire à une hypovolémie et à une hypotension (dengue avec syndrome de choc) souvent compliquée par des hémorragies internes sévères. Le taux de mortalité de la dengue hémorragique peut atteindre jusqu'à 10 % sans thérapie, mais est ≤ 1 % dans la plupart des centres ayant une expérience thérapeutique (WHO technical Guide, 1986. Dengue haemorrhagic fever: diagnosis, treatment and control, p1-2. World Health Organization, Geneva, Switzerland).

Le diagnostic de routine en laboratoire de la dengue est basé sur l'isolation du virus et/ou la détection d'anticorps spécifiques du virus de la dengue.

La dengue est la deuxième maladie infectieuse tropicale la plus importante après la malaria, plus de la moitié de la population mondiale vivant dans des zones à risque de transmission épidémique. Chaque année, les cas de dengue sont estimés à 50-100 millions, les cas de patients hospitalisés pour une dengue hémorragique à 500 000, et le nombre de morts à 25 000. La dengue est endémique en Asie, dans le Pacifique, en Afrique, en Amérique latine et dans les Caraïbes. Plus de 100 pays tropicaux sont endémiques pour les infections par le virus de la dengue et la dengue hémorragique a été documentée dans 60 de ces pays (Gubler, 2002, TRENDS in Microbiology. 10:100-103 ; Monath, 1994, Proc. Natl. Acad. Sci.; 91: 2395-2400). Un certain nombre de facteurs bien décrits semblent être impliqués dans la dengue : la croissance de la population ; une urbanisation non planifiée et non contrôlée, en particulier en association avec la pauvreté ; une augmentation des voyages aériens ; le manque de contrôle effectif des moustiques et la détérioration des infrastructures sanitaires et de santé publique (Gubler, 2002, TRENDS in Microbiology. 10: 100-103). Les voyageurs et expatriés sont de plus en plus alertés contre la dengue (Shirtcliffe et al., 1998, J. Roy. Coll. Phys. Lond.; 32: 235-237). La dengue a constitué une des principales causes de maladies fébriles au sein les troupes américaines au cours de déploiements dans des zones tropicales endémiques pour la dengue (DeFraites et al., 1994, MMWR 1994; 43: 845-848).

Les virus sont maintenus dans un cycle qui implique les humains et *Aedes aegypti*, un moustique domestique piquant le jour, qui préfère s'alimenter sur l'homme. L'infection chez l'homme est initiée par l'injection du virus au cours du repas de sang d'un moustique *Aedes aegypti* infecté. Le virus salivaire est déposé principalement dans les tissus extravasculaires. La première catégorie de cellules infectées après inoculation sont les cellules dendritiques, qui migrent ensuite vers les ganglions lymphatiques (Wu et al., 2000, Nature Med.; 7:816-820). Après une réplication initiale dans la peau et dans les ganglions lymphatiques, le virus apparaît dans le sang au cours de la phase fébrile aiguë, généralement pendant 3 à 5 jours.

Les monocytes et les macrophages sont, avec les cellules dendritiques, parmi les premières cibles du virus de la dengue. La protection contre une réinfection homotypique est complète et dure probablement toute la vie, mais une protection croisée entre les différents types de dengue dure moins de quelques semaines à quelques mois (Sabin, 1952, Am. J. Trop. Med. Hyg.; 1: 30-50). En conséquence, un sujet peut subir une infection avec un sérotype différent. Une seconde infection par la dengue est en théorie un facteur de risque de développer une maladie dengue sévère.

Cependant, la dengue hémorragique est multifactorielle : ces facteurs incluent la souche du virus impliqué, ainsi que l'âge, le statut immun et la prédisposition génétique du patient. Deux facteurs jouent un rôle majeur dans la survenue de la dengue hémorragique: une réplication virale rapide avec une virémie élevée (la sévérité de la maladie étant associée au niveau de la virémie ; Vaughn et al., 2000, J. Inf. Dis.; 181: 2-9) et une réponse inflammatoire importante avec la libération de niveaux élevés de médiateurs inflammatoires (Rothman and Ennis, 1999, Virology; 257: 1-6). Il n'y a aucun traitement spécifique contre la dengue. Le traitement de la fièvre dengue est symptomatique avec un alitement, un contrôle de la fièvre et de la douleur avec des antipyrétiques et des analgésiques, et une prise de boisson adéquate. Le traitement de la dengue hémorragique nécessite l'équilibrage des pertes de liquide, le remplacement des facteurs de coagulation et la perfusion d'héparine.

Les mesures de prévention reposent actuellement sur le contrôle du vecteur et des mesures de protection personnelle qui sont difficiles à mettre en oeuvre et coûteuses. Aucun vaccin contre la dengue n'est approuvé pour le moment. Etant donné que les quatre sérotypes de la dengue sont en circulation dans le monde et comme ils ont été rapportés comme étant impliqués dans des cas de fièvre hémorragique dengue, la vaccination devrait idéalement conférer une protection contre les quatre sérotypes du virus de la dengue.

Lors de l'immunisation par un vaccin tétravalent, il peut arriver que la réponse soit induite de façon prépondérante contre seulement un ou au plus 3 serotypes. Il existe donc un besoin d'une méthode permettant de réduire les interférences entre les différents sérotypes et permettant d'induire des anticorps neutralisant contre les 4 sérotypes de la dengue.

Les inventeurs ont mis en évidence qu'il est possible de générer une réponse immune comprenant des anticorps neutralisant les 4 sérotypes, lorsque la formulation vaccinale devant induire une réponse contre les 4 serotypes est administrée après une primo-immunisation par un vaccin vivant atténué d'un serotype seulement, la deuxième immunisation étant mise en oeuvre 30 jours à 12 mois après la première administration.

Les inventeurs ont en particulier montré qu'une immunisation tetravalente DEN-1,2,3,4 suivant une immunisation monovalente DEN-2 induit des réponses contre les quatre sérotypes chez tous les singes immunisés. A l'inverse, une immunisation tétravalente seule n'a permis d'induire une réponse satisfaisante que contre deux sérotypes sur 4, même après rappel.

La réponse immune générée par la méthode selon la présente invention est donc plus importante quantitativement et qualitativement (couvre tous les sérotypes).

La présente invention est telle que définie par les revendications.

Selon un premier objet, la présente description concerne donc une méthode permettant d'induire une réponse en anticorps neutralisants contre les 4 sérotypes de la dengue chez un patient, comprenant :

(a) une première administration d'un vaccin monovalent comprenant un virus vaccinal de la dengue d'un premier sérotype dans laquelle ledit virus vaccinal utilisé lors de la première administration (a) est sélectionné dans le groupe constitué des souches VDV1 et VDV2 ,

(b) une deuxième administration d'un vaccin tétravalent comprenant des virus vaccinaux des 4 serotypes de la dengue, et

dans laquelle la deuxième administration (b) est mise en oeuvre au moins 30 jours à au plus 12 mois après la première administration (a).

Selon un autre mode de réalisation particulier de la méthode selon la description, les dits virus vaccinaux utilisés dans le vaccin tétravalent sont sélectionnés dans le groupe constitué des Chimerivax™ DEN-1, 2, 3 et 4.

Selon un autre mode de réalisation particulier de la méthode selon la description, la quantité de virus vaccinaux de la dengue de sérotypes 1, 2, 3 et 4 est comprise dans une gamme allant de $10^3$ à $10^6$ $DICC_{50}$.

Selon un autre mode de réalisation particulier de la méthode selon la description, le vaccin monovalent comprend $10^4$ $DICC_{50}$ de VDV1 ou VDV2 et le vaccin tétravalent comprend $10^5$ $DICC_{50}$ de Chimerivax™ DEN-1, 2, 3 et $10^3$ $DICC_{50}$ de Chimerivax™ DEN-4.

Selon un autre mode de réalisation de la méthode selon la description, la deuxième administration (b) est mise en oeuvre 30 à 60 jours après la première administration (a).

La présente invention a également pour objet un kit d'immunisation contre le virus de la dengue comprenant un boitier contenant au moins (a) un premier récipient renfermant un composition ou vaccin monovalent comprenant un virus vaccinal sélectionné dans le groupe constitué des souches VDV1 et VDV2, (b) un deuxième récipient renfermant une composition ou vaccin tétravalent comprenant des virus vaccinaux des 4 sérotypes de la dengue.

Selon un mode de réalisation, le kit selon l'invention comprend au moins:

(a) un premier récipient contenant un vaccin monovalent comprenant un virus vaccinal VDV1 ou VDV2

(b) un deuxième récipient contenant un vaccin tétravalent comprenant les 4 Chimerivax™ DEN-1, 2, 3 et 4.

Selon un mode de réalisation particulier ; le kit selon l'invention comprend un vaccin monovalent comprenant $10^4$ $DICC_{50}$ de VDV1 ou VDV2 et un vaccin tétravalent comprenant $10^5$ $DICC_{50}$ de Chimerivax™ DEN-1, 2, 3 et $10^3$ $DICC_{50}$ de Chimerivax™ DEN-4.

L'invention va être décrite plus en détails dans la description qui suit.

*Définitions*

Les « virus de la dengue » ou « DEN » sont des virus à ARN simple-brin, positif, appartenant au genre Flavivirus de la famille des *flaviviridae.* L'ARN génomique contient une coiffe de type I à l'extrémité 5' mais est dépourvu de queue poly-A à l'extrémité 3'. L'organisation génomique est constituée des éléments suivants: région non codante (NCR) 5', protéines structurales (capside (C), pré-membrane/membrane (prM/M), enveloppe (E)) et protéines non structurales (NS1-NS2A-NS2B-NS3-NS4A-NS4B-NS5) et NCR 3'. L'ARN viral génomique est associé aux protéines de la capside pour former une nucléocapside. Comme pour les autres flavivirus, le génome viral DEN code une région codante ininterrompue qui est traduite en une polyprotéine unique.

Par « virus vaccinal de la dengue» on entend dans le cadre de la présente invention, toute forme virale du virus de la dengue capable d'induire une réponse immune spécifique comprenant des anticorps neutralisants, de préférence toute forme virale du virus de la dengue utilisable dans le cadre d'un programme d'immunisation chez l'homme contre une infection par un virus de la dengue. Par virus vaccinaux de la dengue on entend donc les virus inactivés, les virus atténués, ou les protéines recombinantes telles que la protéine d'enveloppe du virus de la dengue.

Un virus vaccinal est considéré comme « inactivé » s'il ne se réplique plus sur des cellules permissives.

Un virus vaccinal est considéré comme « atténué » si après croissance à 37°C ou 39°C sur cellule hépatique Huh-7, VERO et/ou C6/36, ledit virus vaccinal présente un titre maximal inférieur d'au moins 10 fois au titre maximal obtenu avec la souche parentale sauvage dans les mêmes conditions de culture et tel que mesuré en utilisant la même méthode de titrage. Un virus vaccinal qui présente une croissance diminuée sur au moins l'un des trois types cellulaires identifiés ci-dessus est donc considéré comme « atténué » dans le cadre de la présente invention.

Un virus vaccinal utilisable chez l'homme présente un rapport bénéfices/risques positif, ledit rapport permet généralement de satisfaire aux exigences réglementaires pour l'obtention d'une autorisation de mise sur le marché. Un virus vaccinal de la dengue utilisé dans le cadre de la présente invention est de préférence un virus atténué de telle sorte qu'il n'induise pas la maladie chez l'homme. Avantageusement, ledit virus vaccinal ne conduit qu'à des effets secondaires au plus d'intensité modérée (i.e. moyenne à faible, voire nulle) chez la majorité des sujets vaccinés tout en conservant sa capacité à induire une réponse anticorps neutralisante.

On peut citer à titre d'exemples non limitatifs, de virus vaccinaux de la dengue utilisable dans le cadre de la présente invention : les virus vaccinaux inactivés, les virus vaccinaux atténués tels que les souches atténuées VDV-1, VDV-2, les souches décrites par exemple dans les demandes : WO02/66621, WO0057904, WO0057908, WO0057909 ; WO0057910, WO02/0950075 et WO02/102828, ou les chimères. Les virus chimères ont la particularité de présenter les caractéristiques des virus atténués tels que définis ci-dessus. Tout virus chimères exprimant la protéine d'enveloppe d'un virus dengue et induisant une réponse immune comprenant des anticorps neutralisant le sérotype dont est issue la protéine d'enveloppe peut donc être utilisé dans le cadre de la présente invention. On peut citer à titre d'exemples non limitatifs : les chimerivax ™dengue tels que décrits par exemple dans la demande de brevet WO 98/37911, les chimères dengue/dengue tels que décrits par exemple dans les demandes de brevets WO9640933 et WO0160847. Le virus vaccinal de la dengue de sérotype 1 peut être par exemple la souche vaccinale VDV1 ou un Chimerivax™ DEN-1, en particulier un virus YF17D/DEN-1, ou encore une souche DEN-1 16007/PDK13. Le virus vaccinal de la dengue de sérotype 2 peut être par exemple la souche vaccinale VDV2 ou un Chimerivax™ DEN-2, en particulier un virus YF17D/DEN-2, ou encore une souche DEN-2 16681/PDK53. Le virus vaccinal de la dengue de sérotype 3 peut être un Chimerivax™ DEN-3, en particulier un virus YF17D/DEN-3. Le virus vaccinal de la dengue de sérotype 4 peut être un Chimerivax™ DEN-4, en particulier un virus YF17D/DEN-4.. On pourra se reporter aux demandes identifiées ici pour un descriptif précis des souches mentionnées et de leur procédé d'obtention.

« VDV » ou « vaccin dengue Vero» désigne une souche virale dengue vivante atténuée adaptée sur cellule Vero (i.e. est capable de se répliquer de façon reproductible à niveau significatif sur cellules Vero) et capable d'induire une réponse humorale spécifique, y compris l'induction d'anticorps neutralisants, chez les primates et en particulier chez l'homme.

« VDV-1 » est une souche obtenue à partir d'une souche sauvage DEN-1 16007 ayant subi 11 passages sur cellules PDK (DEN-1 16007/PDK11) qui a ensuite été amplifiée sur cellules Vero à 32°C, et dont l'ARN a été purifié et transfecté dans des cellules Vero. La souche VDV-1 présente 14 mutations supplémentaires par rapport à la souche vaccinale DEN-1 16007/PDK13 (13 passages sur cellules PDK-Primary Dog Kidney). La souche DEN-1 16007/PDK13, aussi appelée « LAV1 », a été décrite dans la demande de brevet EP1159968 au nom de Mahidol University et a été déposée auprès de la Collection Nationale de Cultures de Microorganismes (CNCM) sous le numéro I-2480. La séquence complète de la souche VDV-1 est donnée à la séquence SEQ ID NO :1. Ladite souche peut être facilement reproduite à partir de ladite séquence. Un procédé de préparation et la caractérisation de la souche VDV-1 a été décrit dans la demande de brevet internationale déposée aux noms de Sanofi-Pasteur et du Center for Disease Control and Prevention sous le numéro WO 2006/134433.

« VDV-2 » est une souche obtenue à partir d'une souche sauvage DEN-2 16681 ayant subi 50 passages sur cellules PDK (DEN-2 16681/PDK50), purifiée par plaque et dont l'ARN a été extrait et purifié avant d'être transfectée dans des cellules Vero. La souche VDV-2 a ensuite été obtenue par purification par plaque et amplification sur cellules Vero. La souche VDV-2 présente 10 mutations supplémentaires par rapport à la souche vaccinale DEN-2 16681/PDK53 (53 passages sur cellules PDK), dont 4 mutations silencieuses. La souche DEN-2 16681/PDK53, aussi appelée « LAV2 », a été décrite dans la demande de brevet EP1159968 au nom de Mahidol University et a été déposée auprès de la Collection Nationale de Cultures de Microorganismes (CNCM) sous le numéro I-2481. La séquence complète de la souche VDV-2 est montrée dans la séquence SEQ ID NO :2. La souche VDV-2 peut être facilement reproduite à partir de ladite séquence. Un procédé de préparation et la caractérisation de la souche VDV-2 a été décrit dans la demande de brevet internationale déposée aux noms de Sanofi-Pasteur et du Center for Disease Control and Prevention sous le numéro WO 2006/134433.

Les souches VDV 1 et 2 sont préparées par amplification sur cellules Vero. Les virus produits sont récoltés et clarifiés des débris cellulaires par filtration. L'ADN est digéré par traitement enzymatique. Les impuretés sont éliminées par ultrafiltration. Les titres infectieux peuvent être augmentés par une méthode de concentration. Après ajout d'un stabilisant, les souches sont stockées sous forme lyophilisée ou congelée avant utilisation puis reconstituées extemporanément.

Par « ChimeriVax™ dengue» ou « CYD » on désigne un virus de la fièvre jaune (YF) chimère qui comprend le squelette d'un virus YF dans lequel les séquences codant pour les protéines de pré-membrane et d'enveloppe ont été remplacée par celles d'un virus DEN. On appelle ainsi « CYD-1 ou CYD DEN1 » un virus YF chimère contenant les séquences prM et E d'une souche dengue sérotype 1(DEN-1). On appelle « CYD-2 ou CYD DEN2» un virus YF chimère contenant les séquences prM et E d'une souche DEN-2. On appelle « CYD-3 ou CYD DEN3» un virus YF chimère contenant les séquences prM et E d'une souche DEN-3. On appelle «CYD-4 ou CYD DEN4» un virus YF chimère contenant les séquences prM et E d'une souche DEN-4. La préparation de ces ChimeriVax™ dengue a été décrite en détails dans les demandes de brevet internationales WO 98/37911 et WO 03/101397 auxquelles on peut se reporter pour un descriptif précis de leur procédé de préparation. Les chimères décrits dans les exemples ont été générés par utilisation des séquences prM et E issues des souches DEN 1 PUO359 (TYP1140), DEN2 PUO218, DEN3 PaH881/88 et DEN 4 1228 (TVP 980). Toute souche du virus de la dengue pourrait être utilisée dans le cadre de la présente invention pour la construction des chimères.

De préférence, le virus YF chimère comprend le squelette d'une souche de la fièvre jaune atténuée YF17D (Theiler M, and Smith HH (1937) J Exp. Med 65, p767-786.) (virus YF17D/DEN-1, YF17D/DEN-2, YF17D/DEN-3, YF17D/DEN-4). Des exemples de souches YF17D susceptibles d'être utilisées incluent YF17D204 (YF-Vax®, Sanofi-Pasteur, Swifwater, PA, USA ; Stamaril®, Sanofi-Pasteur, Marcy l'Etoile, France ; ARILVAX™, Chiron, Speke, Liverpool, UK; FLAVIMUN®, Berna Biotech, Bern, Switzerland ; YF17D-204 France (X15067,X15062) ; YF17D-204,234 US (Rice et al., 1985, Science, 229 :726-733), ou encore des souches apparentées YF17DD (Genbank numéro d'accès U17066), YF17D-213 (Genbank numéro d'accès U17067) et les souches YF17DD décrites par Galler et al. (1998, Vaccines 16 (9/10) :1024-1028). Toute autre souche de virus de la fièvre jaune atténuée utilisable chez l'homme peut être utilisée dans le cadre de la présente invention pour la construction des chimères.

Selon un mode de réalisation particulier, les virus vaccinaux sont, pour chaque serotype utilisé dans les différentes administrations, présents dans le vaccin à une quantité de $10^3$ à $10^5$ $DICC_{50}$.

Selon un mode de réalisation particulier, les virus vaccinaux VDV1 ou VDV2 sont présents dans le vaccin monovalent à raison de $10^4$ $DICC_{50}$.

Selon un mode de réalisation particulier les Chimerivax™ DEN-1, 2, 3 sont présents dans le vaccin tétravalent à raison de $10^5$ $DICC_{50}$ et le Chimerivax™ DEN-4 est présent dans le vaccin tétravalent à raison de $10^3$ $DICC_{50}$.

Chaque virus vaccinal dengue monovalent ChimeriVax™ (sérotypes 1, 2, 3 et 4) a été préparé par amplification de chaque sérotype sur cellules Vero. Plus spécifiquement, les quatre virus sont produits séparément sur des cellules Vero adhérentes en milieu sans sérum. La récolte virale, clarifiée des débris cellulaires par filtration, est alors concentrée et purifiée par ultrafiltration et chromatographie pour enlever l'ADN des cellules hôtes. Après ajout d'un stabilisant, les souches vaccinales sont stockées sous forme congelée ou lyophilisée avant utilisation puis reconstituées extemporanément. Le même procédé est appliqué pour les quatre chimères.

Une dose, une composition ou un vaccin est « monovalent » lorsqu'il contient outre un excipient pharmaceutiquement acceptable un virus vaccinal d'un seul sérotype de la dengue. Une dose, une composition ou un vaccin est « tétravalent » lorsqu'il contient des virus vaccinaux des quatre sérotypes de la dengue. Les compositions multivalentes sont obtenues par simple mélange des compositions monovalentes.

Par « patient », on désigne une personne (enfant ou adulte) susceptible d'être infectée par la dengue, en particulier une personne à risque d'infection comme par exemple une personne voyageant dans des régions où la dengue est présente ou un habitant de ces régions. Ce terme englobe donc les personnes naïves ainsi que les personnes non-naïves pour le virus de la dengue.

*Immunisation tétravalente suivant une primo-immunisation monovalente*

Selon un premier aspect, la présente description a donc pour objet une méthode d'immunisation contre le virus de la dengue.
Les inventeurs ont en effet montré en particulier que l'administration des 4 sérotypes 30 jours à 12 mois après la première administration d'un vaccin monovalent permet d'obtenir une protection efficace contre les 4 sérotypes. La méthode selon la présente description présente donc un intérêt tout particulier dans le cadre d'une stratégie d'immunisation contre la dengue.
Selon la présente invention, la première immunisation est pratiquée avec un vaccin monovalent comprenant la souche VDV1 ou VDV2, de préférence la souche VDV2, la deuxième administration étant réalisée avec l'ensemble des 4 sérotypes vaccinaux.
Des virus vaccinaux vivants atténués sont utilisés dans la deuxième administration, de préférence des virus chimères exprimant des antigènes des quatre sérotypes du virus de la dengue, en particulier des Chimerivax™ DEN1, 2, 3 et 4.
Selon des modes de réalisation particuliers, la présente invention couvre donc les schémas suivants :

- (a) VDV1 (b) CYD DEN-1, 2, 3 et 4
- (a) VDV2 (b) CYD DEN-1, 2, 3 et 4.

On entend par « vaccin ou composition » dans le cadre de la présente invention une composition comprenant une « quantité immunoefficace » du virus vaccinal de la dengue, c'est-à-dire une quantité suffisante de virus vaccinal de la dengue pour induire une réponse immune spécifique comprenant des anticorps neutralisants, qui peut être mise en évidence par exemple par le test de séroneutralisation tel que décrit ci-dessous dans l'exemple 1. Un sérum est considéré comme positif pour la présence d'anticorps neutralisants lorsque le titre d'anticorps neutralisants ainsi déterminé est supérieur ou égal à 1 :10 (unité : 1/dilution).
Les quantités de souche vaccinales sont exprimées communément en termes d'unité formant des plages virales (PFU) ou de dose infectant 50% de la culture tissulaire ou encore de dose infectant 50% de la culture cellulaire ($DICC_{50}$) Par exemple, les compositions selon l'invention peuvent contenir de 10 à $10^6$ $DICC_{50}$, en particulier de $10^3$ à $10^5$ $DICC_{50}$ de virus vaccinai dengue de sérotype 1, 2, 3 ou 4 pour une composition monovalente ou tétravalente. Ainsi dans les compositions ou utilisation selon l'invention, les doses de virus vaccinaux de la dengue de sérotype 1, 2, 3 et 4 sont préférentiellement comprises chacune dans une gamme allant de 10 à $10^6$ $DICC_{50}$, tels que 10, $10^2$, $10^3$, $10^4$, $10^5$ ou $10^6$ $DICC_{50}$ en particulier dans une gamme allant de $10^3$ à $10^5$ $DICC_{50}$. Les virus vaccinaux peuvent être utilisés à des doses identiques ou différentes, qui peuvent être ajustées en fonction de la nature du virus vaccinal utilisé et de l'intensité de la réponse immunitaire obtenue.
Selon un mode de réalisation particulier de la méthode selon la présente description, les quantités de virus vaccinaux vivants atténués dans les compositions ou vaccins monovalents et tétravalents sont de $10^3$ à $10^5$ $DICC_{50}$. Selon un mode de réalisation particulier le vaccin monovalent comprend $10^4$ $DICC_{50}$ de VDV1 ou VDV2, de préférence VDV2. Selon un mode de réalisation particulier le vaccin tétravalent comprend $10^5$ $DICC_{50}$ des Chimerivax™ DEN-1, 2, 3 et 4. Selon un mode de réalisation avantageux, le vaccin tétravalent comprend $10^5$ $DICC_{50}$ des Chimerivax™ DEN-1, 2 et 3 et $10^3$ $DICC_{50}$ de Chimerivax™ DEN-4 .
Dans le cadre de la présente invention, la deuxième administration (b) est mise en oeuvre 30 jours à au plus 12 mois après l'administration (a). Selon un mode de réalisation avantageux, la deuxième administration est mise en oeuvre de 30 jours à 60 jours après la première administration (a).
La réponse anticorps neutralisante est avantageusement durable, c'est-à-dire qu'elle peut être détectée dans le sérum jusqu'à au moins 6 mois, après la deuxième administration.
Les virus vaccinaux sont administrés sous forme de compositions ou vaccins qui peuvent être préparés selon n'importe quelle méthode connue de l'homme du métier. Habituellement, les virus, généralement sous forme lyophilisée, sont mélangés avec un excipient pharmaceutiquement acceptable, tel que de l'eau ou une solution saline tamponnée au phosphate, des agents mouillants ou stabilisants. Par « excipient pharmaceutiquement acceptable », on entend tout solvant, milieu de dispersion, charge etc, qui ne produit pas de réaction secondaire, par exemple allergique, chez l'humain ou l'animal. L'excipient est sélectionné en fonction de la forme pharmaceutique choisie, de la méthode et de la voie d'administration. Des excipients appropriés, ainsi que les exigences en matière de formulation pharmaceutique, sont décrites dans « Remington : The Science & Practice of Pharmacy », qui représente un ouvrage de référence dans le domaine.
De préférence, les compositions vaccinales sont préparées sous forme injectable, et peuvent correspondre à des solutions liquides, des suspensions ou des émulsions. Les compositions peuvent en particulier comprendre une solution aqueuse tamponnée de manière à maintenir un pH compris entre environ 6 et 9 (comme déterminé avec un pH-mètre à température ambiante).

Bien qu'il ne soit pas nécessaire de rajouter un adjuvant, Les compositions peuvent néanmoins comprendre un tel composé, c'est-à-dire une substance qui augmente, stimule, ou renforce, la réponse immunitaire cellulaire ou humorale induite par le virus vaccinal administré simultanément. L'homme de l'art est à même de sélectionner parmi les adjuvants classiquement utilisés dans le domaine vaccinal, un adjuvant qui puisse être approprié dans le cadre de la présente invention.

Les compositions ou vaccins selon l'invention peuvent être administrés selon n'importe quelle voie utilisée habituellement en vaccination, par exemple la voie parentérale (notamment ' intradermique, sous-cutanée, ou intramusculaire), avantageusement par voie sous-cutanée. De préférence, les compositions ou vaccin sont des compositions injectables administrées par voie sous-cutanée, avantageusement au niveau de la région du deltoïde gauche ou du deltoïde droit.

Le volume de composition ou vaccin administré dépend de la voie d'administration. Pour des injections sous-cutanées, le volume est généralement compris entre 0,1 et 1,0 ml, de préférence environ 0,5 ml.

La période optimale pour l'administration de la totalité des sérotypes 1 à 4, est d'environ 1 à 3 mois avant l'exposition au virus de la dengue. Les vaccins peuvent être administrés en tant que traitement prophylactique d'une infection par un virus de la dengue chez les adultes et les enfants. Les populations ciblent incluent donc des personnes qui peuvent être naïves (i.e. non préalablement immunisés) ou non-naïves vis-à-vis du virus de la dengue.

Des administrations de rappel des virus vaccinaux de la dengue de sérotypes 1 à 4 peuvent en outre être mises en oeuvre par exemple entre 6 mois et 10 ans, par exemple 6 mois, 1 ans, 3 ans, 5 ans ou 10 ans après l'administration de la deuxième administration(b) selon l'invention. Les administrations de rappel vont être mises en oeuvre avantageusement en utilisant les mêmes compositions ou vaccins (i.e. les mêmes virus vaccinaux) et de préférence dans les mêmes conditions d'administrations (sites anatomiques et voies d'administration) que celles utilisées pour la 2$^{éme}$ s administration (b).

Les phénomènes d'interférence peuvent s'expliquer par la dominance d'un ou plusieurs sérotypes par rapport à d'autres et sont donc indépendants de la technologie utilisée pour la fabrication du candidat vaccin (ex VDV ou Chimerivax™). La méthode selon la présente description peut donc s'appliquer de façon générale à tout virus vaccinal de la dengue.

La présente description entend donc couvrir également l'utilisation de virus vaccinaux de la dengue pour la fabrication d'un vaccin monovalent et d'un vaccin tétravalent pour une immunisation contre le virus de la dengue dans laquelle le vaccin monovalent comprend un virus vaccinal de la dengue d'un premier sérotype, le vaccin tétravalent comprend des virus vaccinaux des 4 sérotypes de la dengue et dans laquelle le vaccin tétravalent est administré au moins 30 jours à au plus 12 mois après l'administration du vaccin monovalent.

Pour un descriptif des vaccins et conditions utilisables dans le cadre de l'utilisation selon la présente invention on peut se reporter au descriptif qui en est donné en relation avec la méthode d'immunisation selon la description.

Selon un autre aspect, la présente invention a pour objet un kit d'immunisation contre les quatre sérotypes du virus de la dengue. Le kit selon la présente invention comprend les compositions ou vaccins tels que définis ci-dessus en relation avec la méthode d'immunisation proposée. Le kit selon l'invention comprend donc un boitier renfermant les différents récipients contenant les compositions ou vaccins et avantageusement une brochure explicative renfermant les informations utiles pour l'administration desdites compositions ou vaccins.

Selon un mode de réalisation, la présente invention a donc pour objet un kit d'immunisation contre le virus de la dengue un boitier contenant au moins (a) un premier récipient renfermant un vaccin monovalent comprenant un virus vaccinal d'un premier sérotype de la dengue sélectionné dans le groupe constitué des souches VDV1 et VDV2, et (b) un deuxième récipient renfermant un vaccin tétravalent comprenant des virus vaccinaux des 4 sérotypes de la dengue.

Pour un descriptif des vaccins, compositions ou virus vaccinaux de la dengue utilisables dans le kit selon l'invention, on peut se référer à la description qui en est donnée ci-dessus en relation avec la méthode d'immunisation selon la description.

Selon un mode de réalisation particulier, le kit selon l'invention comprend au moins:

(a) un premier récipient contenant un vaccin monovalent comprenant un virus vaccinal VDV1 ou VDV2 et
(b) un deuxième récipient contenant un vaccin tétravalent comprenant les 4 Chimerivax™ DEN-1, 2, 3 et 4.

Selon un mode de réalisation particulier, le kit selon l'invention comprend au moins un vaccin monovalent comprenant $10^4$ $DICC_{50}$ de VDV1 ou VDV2 et un vaccin tétravalent comprenant $10^5$ $DICC_{50}$ de Chimerivax™ DEN-1, 2, 3 et $10^3$ $DICC_{50}$ de Chimerivax™ DEN-4.

Les kits selon l'invention peuvent contenir un exemplaire unique ou plusieurs exemplaires des récipients tels que décrits ci-dessus.

Si les vaccins utilisés se présentent sous forme lyophilisée, le kit comprendra avantageusement au moins un récipient supplémentaire contenant le diluant permettant de reconstituer une dose vaccinale injectable. Tout diluant pharmaceutiquement acceptable peut être utilisé pour ce faire, classiquement, de l'eau ou une solution aqueuse tamponnée au phosphate.

L'invention est illustrée par l'exemple suivant.

Exemple 1 : Immunisation contre les 4 sérotypes du virus de la dengue par injection successive d'une composition monovalente suivie par une composition tétravalente chez le singe.

La virémie et l'immunogénicité ont été testées dans un modèle singe. La virémie, en particulier, a été identifiée comme l'un des facteurs associés avec la virulence et la sévérité de la maladie chez les hommes et constitue donc un paramètre important à prendre en considération. L'immunogénicité est quant à elle un paramètre clé dans le cadre de l'évaluation de la protection conférée.

1.1 Matériels et méthodes :

Les expériences chez les singes ont été menées selon les Directives européennes relatives à l'expérimentation animale. Les immunisations ont été effectuées chez des singes cynomolgus (*Macaca fascicularis*) originaires de Mauritanie. Les singes ont été mis en quarantaine pendant six semaines avant immunisation.

Les singes ont été immunisés par voie sous-cutanée dans les bras avec 0,5 ml de composition vaccinale. Après une légère anesthésie avec de la kétamine (Imalgene, Merial), du sang a été collecté par ponction au niveau des veines inguinale ou saphène. Aux jours 0 et 28 suivant chaque immunisation, 5 ml de sang ont été échantillonnés pour évaluer les réponses anticorps, alors qu'entre les jours 2 et 10, 1 ml de sang était échantillonné pour évaluer la virémie. Le sang était collecté sur la glace et conservé sur la glace jusqu'à séparation du sérum. Pour ce faire, le sang a été centrifugé pendant 20 minutes à 4°C et le sérum collecté stocké à -80°C jusqu'au moment des tests.

Mesure de la virémie

Les virémies post vaccinales ont été suivies par RT-PCT quantitative en temps réel (qRT-PCR). Deux jeux d'amorces et de sondes localisées dans le gène NS5 de des souches DEN1 et DEN2 ont été utilisés pour quantifier l'ARN de VDV-1 et VDV-2 respectivement. Un troisième jeu de 2 amorces et 1 sonde localisées dans le gène NS5 du virus YF a été utilisé pour quantifier l'ARN du CYD. Enfin, 4 jeux d'amorces et de sondes spécifiques des différents sérotypes CYD, localisées à la jonction des gènes E (DEN) / NS1 (YF) ont été utilisés pour identifier le sérotype dans les échantillons positifs pour l'ARN NS5 YF (voir aussi tableau I). 7 plasmides contenant, sous le contrôle du promoteur T7, la région ciblée par chaque PCR, ont été transcrits *in vitro* pour générer une série d'ARN synthétiques qui ont été inclus comme référence interne dans chaque essai de RT-PCT. Ces ARN synthétiques ont été dosés par spectrophotométrie, la quantité d'ARN obtenue a été convertie en nombre de copies d'ARN et exprimée en GEQ (équivalents génomiques).

0,140 ml de sérum de singe a été extrait à l'aide du kit d'extraction d'ARN « Nucleospin 96 virus™ » de Macherey Nagel, selon les instructions du fabriquant, puis l'ARN purifié a été élué avec 0,140 ml (0,090 ml, puis 0,05 ml) d'eau exempte de RNase. Pour éviter des cycles répétés de congélation/décongélation, une première quantification a été réalisée immédiatement après l'extraction sur 5 $\mu$l de ladite préparation d'ARN. Le volume restant a été congelé à 70°C.

Les mélanges réactionnels contenaient, en plus des composants du kit de quantification RT-PCR « Qiagen Qauntitect™probes » (Qiagen), 10 picomoles de chaque amorce, 4 picomoles de chaque sonde et 5 $\mu$l d'ARN, dans un volume total de 25 $\mu$l. Dans le cas des ARN à tester, 5 $\mu$l de la préparation purifiée ont été directement introduits dans le mélange réactionnel, sans étape de dilution préalable. Les ARN synthétiques ont été dilués au 1/10 dans de l'eau exempte de RNAse, et 7 dilutions contenant approximativement 10 à $10^6$ GEQ dans 5 $\mu$l ont été quantifiées en parallèle afin de générer la courbe étalon.

Les réactions de quantification ont été réalisées par l'appareil ABIPrism 700™ d'Applied Biosystem, en utilisant le programme suivant : 50°C/30 min, 95°C/15 min, puis 40 cycles de 95°C/15 sec-60°C/60 sec.

La limite de quantification de l'ARN viral dans ce test est de 2,9 à 3,3 $\log_{10}$GEQ/ml (800 à 2000 GEQ/ml; 4 à 10 GEQ/réaction), selon les cibles PCR (déviation standard : +/-0,3 $\log_{10}$)

La corrélation entre le titre infectieux et la quantification d'ARN viral a été établie parallèlement aux essais, par analyse de 0,140 ml d'échantillons de sérums de singe négatifs (DO) dans lesquels on a ajouté une quantité connue de particules infectieuses des virus ayant servi à l'immunisation (CYD ou VDV). Lesdits sérums contrôles ont été préparés à deux dilutions contenant environ 1 PFU et environ 100 PFU dans 5 $\mu$l (2.3 and 4.3 $\log_{10}$PFU/ml, respectivement).

Dans les tests utilisés dans les exemples la corrélation entre GEQ et PFU est la suivante :Ratio GEQ/PFU de 2.7 $\log_{10}$ (soit : 1 PFU = 500 GEQ) pour les sera positifs en YF ou CYDs. Ratio GEQ/PFU de 2.5 $\log_{10}$ (soit : 1 PFU = 320 GEQ) pour les sera positifs en VDV1 ou VDV2.

Les Limites de quantification étant < 3.3 $\log_{10}$GEQ/ml (soit : <4 PFU/ml) pour les qRT-PCR YF et CYDs et <

2.9 $log_{10}$GEQ/ml (soit: < 2.5 PFU/ml) pour les qRT-PCR VDV1 et VDV2.

Les amorces et sondes utilisées sont données dans le tableau 1 ci-dessous dans lequel sont listés dans l'ordre pour chaque essai les amorces sens et anti-sens et la sonde.

## Tableau 1

| | | | sequence |
|---|---|---|---|
| YF | YF-NS5 sens | | 5' GCACGGATGTAACAGACTGAAGA (23 bases) |
| | YF NS5 anti | | 5' CCAGGCCGAACCTGTCAT (18 bases) |
| | YF-NS5 | | 5' Fam- CGACTGTGTGGTCCGGCCCATC -Tamra (22 bases) |
| CYD1 spe | CYD1- | sens | 5' CAT TGC AGT TGG CCT GGT AA (20 b) |
| | CYD1- | antis | 5' CTT TGG CAA GAG AGA GCT CAA GT (23 b) |
| | CYD1- | | 5' Fam-CCG ATC AAG GAT GCG CCA TCA-Tamra (21 b) |
| CYD2 spe | CYD2- | sens | 5' GTG GGA GTC GTG ACG CTG TA (20 b) |
| | CYD2- | anti | 5' GTT GAT GGC GCA TCC TTG ATC (21 b) |
| | CYD2 | | 5' Fam-TGG GAG TTA TGG TGG GCG CCG-Tamra (21 b) |
| CYD3 spe | CYD3- | sens | 5' AAA ACA CTT CCA TGT CAT TTT CAT G (25b) |
| | CYD3- | anti: | 5' GTT GAT GGC GCA TCC TTG ATC (21 b) |
| | CYD3- | | 5'Fam-TGCGATAGGAATTATCACACTCTATCTGGGAGC-Tamra (33b) |
| CYD4 spe | CYD4- | sens | 5' CTT AGT ATT GTG GAT TGG CAC GAA (24 b) |
| | CYD4- | anti: | 5' GCG CCA ACT GTG AAA CCT AGA (21 b) |
| | CYD4- | | 5'-Fam-AGAAACACTTCAATGGCAATGACGTGCAT-Tamra (29 b) |
| VDV1 spe | VDV1-NS5 sens | | 5' TCG CAA CAG CCT TAA CAG C (19 b) |
| | VDV1-NS5 anti | | 5' ACT ATC TCC CTC CCA TCC TTC (21 b) |
| | VDV1-NS5 | | 5' Fam-TTC ACA CCA CTT CCA C-MGB/NFQ (16 b) |
| VDV2 spec | VDV2-NS5 sens | | 5' AAT GAC AGA CAC GAC TCC (18 b) |
| | VDV2-NS5 anti: | | 5' CCC AAA ACC TAC TAT CTT CAA C (22 b) |
| | VDV2-NS5 | | 5' Fam-TGG AAG TCG GCA CGT GA-MGB/NFQ (17 b) |

Mesure des anticorps neutralisants (test de seroneutralisation) (SN50))

Classiquement, la mesure des anticorps Dengue est établie à l'aide du test PRNT50 (test de neutralisation par réduction à 50% du nombre de PFU). Ce test étant lourd et consommateur de matériel, nous avons développé le test SN50, basé sur la réduction à 50 % du nombre d'unités mesurées en test DICC50.

Dans une plaque de 96 puits, 0,120 ml de chaque sérum décomplémenté est ajouté à 0,480 ml de diluant (ISCOVE 4% SVF) par puits. Des dilutions en série d'un facteur 6 sont réalisées par transfert de 0,150 ml de sérum dans 0,450 ml de diluant. 450 $\mu$l de dilution virale à 2,7 $log_{10}$ DICC50/ml sont ajoutés dans chaque puits de façon à obtenir 25 DICC50/puits. La plaque est incubée à 37°C pendant 1 heure. 0.1 ml de chaque dilution est ensuite distribué dans 6 puits d'une plaque de 96 puits dans laquelle des cellules VERO ont été ensemencées 3 jours avant le début de l'expérience à une densité de 8000 cellules/puits, dans 0.1 ml de milieu ISCOVE 4% SVF,. Après 6 jours d'incubation à 37°C, en présence de 5% de $CO_2$, les cellules sont fixées à l'aide d'un mélange éthanol/acétone (70/30) à 4°C pendant 15 minutes, puis lavées à 3 reprises dans du PBS et incubées pendant 1h à 37°C en présence de 0.05 ml d'une dilution au 1/2000 d'un anticorps monoclonal anti-flavivirus (mAb 4G2-obtenu à partir d'un hybridome ATCC H-B112). Les plaques sont ensuite lavées à 2 reprises et incubées 1H à 37°C en présence de 0.05 ml d'une dilution au 1/1000 d'une IgG anti-souris conjuguée à la phosphatase alcaline. Les plages de lyse sont révélées par addition de 0.05 ml d'un substrat coloré : BCIP/NBT. Les titres en anticorps neutralisants sont calculés en utilisant la formule de Karber telle que définie ci-dessous :

$$\log_{10}SN50 = d + f/N\ (X + N/2),$$

Dans laquelle:

- d : représente la dilution conduisant à 100% de neutralisation (soit 6 réplicats négatifs c'est-à-dire ne présentant pas de signe d'infection)
- f: représente le facteur de dilution en $log_{10}$ (e.g. facteur de dilution de 1:4, f = 0,6)

N: représente le nombre de réplicats / dilution (N=6)
X: nombre total de puits ne présentant aucun signe d'infection, à l'exception de la dilution d.

La limite de détection virale est de 10 SN50 (i .e. 1.0-$log_{10}$SN50).
Les souches virales qui ont été utilisées pour la neutralisation sont les souches DEN1 16007, DEN2 16681, DEN3 16562 or DEN4 1036.
Pour les contrôles, les dilutions virales initiales ont été re-titrées.
La corrélation entre le titre neutralisant mesuré en test SN50 et le titre neutralisant mesuré classiquement en test PRNT50 est : $log_{10}$PRNT50 = $log_{10}$SN50 + 0,2.

1.2 Evaluation des immunisations simultanées

2 groupes de 4 singes d'âge et poids équivalents ont été immunisés. (voir tableau 2)
L'immunisation a été effectuée par voie sous-cutanée dans le bras avec une aiguille 23G1, avec une quantité de $10^5$ $DICC_{50}$ pour chaque sérotype CYD DEN 1 à 4 pour le vaccin tétravalent et avec une quantité de dose $10^4$ $DICC_{50}$ pour le VDV-2 monovalent.

Tableau 2 : Composition des groupes et protocole d'immunisation

| Singes | | |
|---|---|---|
| Groupes | Immunisations | |
| | J0 | J56 |
| Groupe 1 | VDV 2 monovalent | Chimeri Vax Dengue Tétravalent 1234 |
| Groupe 2 | Chimeri Vax Dengue tétravalent 1234 | Chimeri Vax Dengue Tétravalent 1234 |

Les résultats d'immunogénicité obtenus après une immunisation (J0+28) et deux immunisations (J 56+28) sont présentés dans le tableau 3.
Les résultats de virémie sont donnés dans le tableau 4.

**Tableau 3 :** titre neutralisant SN50 (unités 1/dil)

| **Singes** | | | **J0+28** | | | | **J56+28** | | | |
|---|---|---|---|---|---|---|---|---|---|---|
| **ID** | **Immunisations** | | **DEN-1** | **DEN-2** | **DEN-3** | **DEN-4** | **DEN-1** | **DEN-2** | **DEN-3** | **DEN-4** |
| | **J0** | **J56** | | | | | | | | |
| AM762 | **VDV2** | **CYD 1234** | **10** | **501** | **-** | **10** | **63** | **1005** | **63** | **200** |
| AM839 | | | - | **802** | - | - | **80** | **1271** | **63** | **504** |
| AM905 | | | **20** | **158** | **-** | **-** | **318** | **1010** | **252** | **506** |
| AN011 | | | **13** | **1005** | **-** | **-** | **252** | **1271** | **319** | **1010** |
| Moyenne géométrique | | | **11** | **503** | ***<10*** | **<10** | **142** | **1131** | **134** | **477** |
| AM496 | **CYD 1234** | **CYD 1234** | **50** | **-** | **16** | **32** | **100** | **40** | **80** | **252** |
| AM645 | | | **-** | **-** | **13** | **31** | **16** | **-** | **-** | **63** |
| AM766 | | | **-** | **-** | **-** | **32** | **20** | **-** | **-** | **80** |
| AM813 | | | **25** | **-** | **-** | **13** | **63** | **13** | **20** | **63** |
| Moyenne géométrique | | | **13** | **<10** | **<10** | **25** | **38** | **11** | **14** | **95** |
| - : titre < 10 | | | | | | | | | | |

Tableau 4 : analyse des virémies (unités :log10 GEQ/mL)

| Groupe | Singe | Primo-immunisation | | | | | | | | | Rappel | | | | | | | | |
|---|---|---|---|---|---|---|---|---|---|---|---|---|---|---|---|---|---|---|---|
| | | J2 | J3 | J4 | J5 | J6 | J7 | J8 | J9 | J10 | J58 | J59 | J60 | J61 | J62 | J63 | J64 | J65 | J66 |
| 1 prime VDV 2 rappel CYD 1,2,3,4 | AM762 | <2,7 | <2,7 | <2,7 | <2,7 | **4,77** | **4,89** | **4,84** | **4,47** | <2,7 | < 3,2 | < 3,2 | < 3,2 | 3,11 | < 3,2 | < 3,2 | 3,65 | 3,59 | < 3,2 |
| | AM839 | **5,11** | **4,51** | **4,19** | **4,19** | **4,56** | **3,69** | <2,7 | <2,7 | <2,7 | 4,98 | 4,86 | 4,43 | 3,79 | < 3,2 | < 3,2 | < 3,2 | < 3,2 | < 3,2 |
| | AM905 | <2,7 | <2,7 | <2,7 | <2,7 | **4,16** | **4,31** | **4,16** | **3,50** | **4,14** | < 3,2 | < 3,2 | 3,09 | 3,42 | 3,39 | < 3,2 | < 3,2 | 4,08 | 4,32 |
| | AN011 | <2,7 | <2,7 | **3,75** | **4,29** | **4,35** | **4,22** | **3,51** | <2,7 | **3,28** | 3,55 | 3,42 | < 3,2 | < 3,2 | 3,42 | 3,37 | 4,67 | 5,09 | 4,97 |
| 2 prime CYD 1,2,3,4 rappel CYD 1,2,3,4 | AM496 | 4,22 | 3,36 | 3,71 | 4,15 | 3,14 | < 3,1 | 3,58 | < 3,1 | < 3,1 | <3,2 | <3,2 | <3,2 | <3,2 | <3,2 | <3,2 | <3,2 | <3,2 | <3,2 |
| | AM645 | ***4,21*** | 3,81 | 2,82 | 3,51 | 3,65 | 3,24 | < 3,1 | 3,47 | 3,44 | <3,2 | <3,2 | <3,2 | <3,2 | <3,2 | <3,2 | <3,2 | <3,2 | <3,2 |
| | AM766 | 3,97 | 3,06 | 3,38 | 4,19 | 3,80 | 3,73 | < 3,1 | < 3,1 | < 3,1 | <3,2 | <3,2 | <3,2 | <3,2 | <3,2 | <3,2 | <3,2 | <3,2 | <3,2 |
| | AM813 | 4,81 | 4,60 | 3,17 | < 3,1 | < 3,1 | < 3,1 | < 3,1 | < 3,1 | < 3,1 | <3,2 | <3,2 | <3,2 | <3,2 | <3,2 | <3,2 | <3,2 | <3,2 | <3,2 |

| |
|---|
| ***CYD1*** |
| *CYD4* |
| **VDV2** |

Brièvement, les résultats peuvent être résumés comme suit :

- Le schéma d'administration selon la présente invention permet d'augmenter qualitativement et quantitativement la réponse anticorps neutralisante qui est obtenue avec un schéma comprenant deux immunisations identiques de vaccin tétravalent.
- Une immunisation CYD-1,2,3,4 pratiquée après une primo-immunisation monovalente VDV2 induit des réponses à des niveaux élevés contre les quatre sérotypes chez tous les singes, contrairement à un schéma comprenant 2 immunisations de vaccin tétravalent.
- La primo-immunisation conduite avec le VDV2 induit comme cela était attendu une réponse dirigée quasi-exclusivement contre le serotype 2, présentant une cross-réactivité de faible intensité contre les serotypes 1 et 4 chez certains animaux.
- Une virémie est observée avec le VDV2 après primo-immunisation, et est causée de manière prédominante par CYD-4 après la deuxième administration (b) (groupe 1). Aucune différence notable n'est observée avec la virémie induite après une primo-immunisation de vaccin tétravalent chez des animaux naïfs (groupe 2). On peut donc en conclure le schéma proposé par l'invention ne favorise pas l'émergence d'une virémie des sérotypes 1, 3 et 4 après la deuxième administration.

Les exemples montrent donc que la méthode d'immunisation selon la présente description améliore l'immunogénicité des virus vaccinaux de la dengue sans altérer la sécurité de ces derniers.

## Revendications

1. Utilisation de virus vaccinaux de la dengue pour la fabrication d'un vaccin monovalent et d'un vaccin tétravalent pour une immunisation contre le virus de la dengue comprenant:

(a) une première administration d'un vaccin monovalent comprenant un virus vaccinal de la dengue d'un premier sérotype dans laquelle ledit virus vaccinal est sélectionné dans le groupe constitué des souches VDV1 dont la séquence complète est donnée dans le SEQ ID NO:1 et VDV2 dont la séquence complète est donnée dans le SEQ ID NO:2,
(b) une deuxième administration d'un vaccin tétravalent comprenant des virus vaccinaux des 4 sérotypes 1 à 4 de la dengue et dans laquelle le vaccin tétravalent est administré au moins 30 jours à au plus 12 mois après l'administration du vaccin monovalent.

**2.** Utilisation d'un virus vaccinal de la dengue d'un premier sérotype dans laquelle ledit virus vaccinal est sélectionné dans le groupe constitué des souches VDV1 dont la séquence complète est donnée dans le SEQ ID NO:1 et VDV2 dont la séquence complète est donnée dans le SEQ ID NO:2, pour la fabrication d'un vaccin monovalent pour usage dans une méthode permettant d'induire une réponse en anticorps neutralisants contre les 4 sérotypes de la dengue chez un patient, comprenant:

(a) une première administration du vaccin monovalent,
(b) une deuxième administration d'un vaccin tétravalent comprenant des virus vaccinaux des 4 sérotypes 1 à 4 de la dengue et dans laquelle le vaccin tétravalent est administré au moins 30 jours à au plus 12 mois après l'administration du vaccin monovalent.

**3.** Utilisation de virus vaccinaux des 4 sérotypes 1 à 4 de la dengue, pour la fabrication d'un vaccin tétravalent pour usage dans une méthode permettant d'induire une réponse en anticorps neutralisants contre les 4 sérotypes de la dengue chez un patient, comprenant:

(a) une première administration d'un vaccin monovalent comprenant un virus vaccinal de la dengue d'un premier sérotype dans laquelle ledit virus vaccinal est sélectionné dans le groupe constitué des souches VDV1 dont la séquence complète est donnée dans le SEQ ID NO:1 et VDV2 dont la séquence complète est donnée dans le SEQ ID NO:2,
(b) une deuxième administration du vaccin tétravalent et dans laquelle le vaccin tétravalent est administré au moins 30 jours à au plus 12 mois après l'administration du vaccin monovalent.

**4.** Utilisation selon l'une quelconque des revendications 1 à 3 dans laquelle les virus vaccinaux utilisés dans le vaccin tétravalent sont les CYD DEN-1, 2, 3 et 4.

**5.** Utilisation selon l'une quelconque des revendications 1 à 4 dans laquelle la quantité de virus vaccinaux de la dengue de sérotypes 1, 2, 3 et 4 est comprise dans une gamme allant de $10^3$ à $10^6$ $DICC_{50}$.

**6.** Utilisation selon l'une quelconque des revendications 1 à 5, dans laquelle le vaccin monovalent comprend $10^4$ $DICC_{50}$ de VDV1 ou VDV2 et le vaccin tétravalent comprend $10^5$ $DICC_{50}$ de CYD DEN-1, 2, 3 et 4.

**7.** Utilisation selon l'une quelconque des revendications 1 à 5, dans laquelle le vaccin monovalent comprend $10^4$ $DICC_{50}$ de VDV1 ou VDV2 et le vaccin tétravalent comprend $10^5$ $DICC_{50}$ de CYD DEN-1, 2, 3 et $10^3$ $DICC_{50}$ de CYD DEN-4.

**8.** Utilisation selon l'une quelconque des revendications 1 à 7, dans laquelle la deuxième administration (b) est mise en oeuvre 30 à 60 jours après la première administration (a).

**9.** Kit d'immunisation contre le virus de la dengue comprenant un boitier contenant au moins:

(a) un premier récipient renfermant un vaccin monovalent comprenant un virus vaccinal sélectionné dans le groupe constitué des souches VDV1 dont la séquence complète est donnée dans le SEQ ID NO:1 et VDV2 dont la séquence complète est donnée dans le SEQ ID NO:2,
(b) un deuxième récipient renfermant un vaccin tétravalent comprenant des virus vaccinaux des 4 sérotypes 1 à 4 de la dengue.

**10.** Kit d'immunisation selon la revendication 9 dans lequel le deuxième récipient contient un vaccin tétravalent comprenant les 4 CYD DEN-1, 2, 3 et 4.

**11.** Kit d'immunisation selon la revendication 10 dans lequel le vaccin monovalent comprend $10^4$ $DICC_{50}$ de VDV1 ou VDV2 et le vaccin tétravalent comprend $10^5$ $DICC_{50}$ de CYD DEN-1, 2, 3 et 4.

12. Kit d'immunisation selon la revendication 10 dans lequel le vaccin monovalent comprend $10^4$ $DICC_{50}$ de VDV1 ou VDV2 et le vaccin tétravalent comprend $10^5$ $DICC_{50}$ de CYD DEN-1, 2, 3 et $10^3$ $DICC_{50}$ de CYD DEN-4.

13. Les virus vaccinaux de la dengue pour usage dans une immunisation contre le virus de la dengue comprenant:

(a) une première administration d'un vaccin monovalent comprenant un virus vaccinal de la dengue d'un premier sérotype dans laquelle ledit virus vaccinal est sélectionné dans le groupe constitué des souches VDV1 dont la séquence complète est donnée dans le SEQ ID NO:1 et VDV2 dont la séquence complète est donnée dans le SEQ ID NO:2,
(b) une deuxième administration d'un vaccin tétravalent comprenant des virus vaccinaux des 4 sérotypes 1 à 4 de la dengue et dans laquelle le vaccin tétravalent est administré au moins 30 jours à au plus 12 mois après l'administration du vaccin monovalent.

14. Un vaccin monovalent comprenant un virus vaccinal de la dengue d'un premier sérotype dans lequel ledit virus vaccinal est sélectionné dans le groupe constitué des souches VDV1 dont la séquence complète est donnée dans le SEQ ID NO:1 et VDV2 dont la séquence complète est donnée dans le SEQ ID NO:2, pour usage dans une méthode permettant d'induire une réponse en anticorps neutralisants contre les 4 sérotypes de la dengue chez un patient, comprenant:

(a) une première administration du vaccin monovalent;
(b) une deuxième administration d'un vaccin tétravalent comprenant des virus vaccinaux des 4 sérotypes 1 à 4 de la dengue et dans laquelle le vaccin tétravalent est administré au moins 30 jours à au plus 12 mois après l'administration du vaccin monovalent.

15. Un vaccin tétravalent comprenant des virus vaccinaux des 4 sérotypes 1 à 4 de la dengue, pour usage dans une méthode permettant d'induire une réponse en anticorps neutralisants contre les 4 sérotypes de la dengue chez un patient, comprenant:

(a) une première administration d'un vaccin monovalent comprenant un virus de la dengue d'un premier sérotype dans laquelle ledit virus vaccinal est sélectionné dans le groupe constitué des souches VDV1 dont la séquence complète est donnée dans le SEQ ID NO:1 et VDV2 dont la séquence complète est donnée dans le SEQ ID NO:2;
(b) une deuxième administration du vaccin tétravalent et dans laquelle le vaccin tétravalent est administré au moins 30 jours à au plus 12 mois après l'administration du vaccin monovalent.

## Patentansprüche

1. Verwendung von Impfviren des Dengue-Fiebers für die Herstellung eines monovalenten Vakzins und eines tetravalenten Vakzins für eine Immunisierung gegen das Virus des Dengue-Fiebers, die Folgendes beinhaltet:

(a) eine erste Verabreichung eines monovalenten Vakzins, umfassend ein Impfvirus des Dengue-Fiebers eines ersten Serotyps, wobei das genannte Impfvirus ausgewählt ist aus der Gruppe bestehend aus Stämmen VDV1, deren komplette Sequenz in der SEQ ID NR:1 angegeben ist, und VDV2, deren komplette Sequenz in der SEQ ID NR:2 angegeben ist,
(b) eine zweite Verabreichung eines tetravalenten Vakzins, das Impfviren der 4 Serotypen 1 bis 4 des Dengue-Fiebers beinhaltet und bei dem das tetravalente Vakzin wenigstens 30 Tage bis höchstens 12 Monate nach der Verabreichung des monovalenten Vakzins verabreicht wird.

2. Verwendung eines Impfvirus des Dengue-Fiebers eines ersten Serotyps, bei dem das genannte Impfvirus ausgewählt ist aus der Gruppe bestehend aus den Stämmen VDV1, deren komplette Sequenz in der SEQ ID NR:1 angegeben ist, und VDV2, deren komplette Sequenz in der SEQ ID NR:2 angegeben ist, für die Herstellung eines monovalenten Vakzins zur Verwendung in einem Verfahren, das die Einleitung einer Antikörperreaktion zulässt, die gegen die 4 Serotypen des Dengue-Fiebers bei einem Patienten neutralisiert, die Folgendes beinhaltet:

(a) eine erste Verabreichung des monovalenten Vakzins,
(b) eine zweite Verabreichung eines tetravalenten Vakzins, das Impfviren der 4 Serotypen 1 bis 4 des Dengue-Fiebers enthält und bei dem das tetravalente Vakzin wenigstens 30 Tage bis höchstens 12 Monate nach der Verabreichung des monovalenten Vakzins verabreicht wird.

**3.** Verwendung von Impfviren der 4 Serotypen 1 bis 4 des Dengue-Fiebers für die Herstellung eines tetravalenten Vakzins für die Verwendung in einem Verfahren, das das Einleiten einer Antikörperreaktion zulässt, die gegen die 4 Serotypen des Dengue-Fiebers bei einem Patienten neutralisiert, die Folgendes beinhaltet:

(a) eine erste Verabreichung eines monovalenten Vakzins, umfassend ein Impfvirus des Dengue-Fiebers eines ersten Serotyps, bei dem das genannte Impfvirus ausgewählt ist aus der Gruppe bestehend aus Stämmen VDV1, deren komplette Sequenz in der SEQ ID NR:1 angegeben ist, und VDV2, deren komplette Sequenz in der SEQ ID NR:2 angegeben ist,
(b) eine zweite Verabreichung des tetravalenten Vakzins, wobei das tetravalente Vakzin wenigstens 30 Tage bis höchstens 12 Monate nach der Verabreichung des monovalenten Vakzins verabreicht wird.

**4.** Verwendung nach einem der Ansprüche 1 bis 3, bei der die in dem tetravalenten Vakzin verwendeten Impfviren CYD DEN-1, 2, 3 und 4 sind.

**5.** Verwendung nach einem der Ansprüche 1 bis 4, bei der die Menge an Impfvirus des Dengue-Fiebers der Serotypen 1, 2, 3 und 4 in einem Bereich von $10^3$ bis $10^6$ $DICC_{50}$ liegt.

**6.** Verwendung nach einem der Ansprüche 1 bis 5, bei der das monovalente Vakzin $10^4$ $DICC_{50}$ VDV1 oder VDV2 enthält und das tetravalente Vakzin $10^5$ $DICC_{50}$ von CYD DEN-1, 2, 3 und 4 enthält.

**7.** Verwendung nach einem der Ansprüche 1 bis 5, bei der das monovalente Vakzin $10^4$ $DICC_{50}$ von VDV1 oder VDV2 enthält und das tetravalente Vakzin $10^5$ $DICC_{50}$ von CYD DEN-1, 2, 3 und $10^3$ $DICC_{50}$ von CYD DEN-4 enthält.

**8.** Verwendung nach einem der Ansprüche 1 bis 7, bei der die zweite Verabreichung (b) 30 bis 60 Tage nach der ersten Verabreichung (a) erfolgt.

**9.** Immunisierungskit gegen das Virus des Dengue-Fiebers, der ein Gehäuse umfasst, das wenigstens Folgendes enthält:

(a) ein erstes Gefäß, das ein monovalentes Vakzin aufnimmt, das ein Impfvirus enthält, ausgewählt aus der Gruppe bestehend aus Stämmen VDV1, deren komplette Sequenz in der SEQ ID NR:1 enthalten ist, und VDV2, deren komplette Sequenz in der SEQ ID NR:2 enthalten ist,
(b) ein zweites Gefäß, das ein tetravalentes Vakzin enthält, das Impfvirus der 4 Serotypen 1 bis 4 des Dengue-Fiebers beinhaltet.

**10.** Immunisierungskit nach Anspruch 9, bei dem das zweite Gefäß ein tetravalentes Vakzin enthält, das die 4 CYD DEN-1, 2, 3 und 4 enthält.

**11.** Immunisierungskit nach Anspruch 10, bei dem das monovalente Vakzin $10^4$ $DICC_{50}$ von VDV1 oder VDV2 beinhaltet und das tetravalente Vakzin $10^5$ $DICC_{50}$ von CYD DEN-1, 2, 3 und 4 beinhaltet.

**12.** Immunisierungskit gemäß Anspruch 10, bei dem das monovalente Vakzin $10^4$ $DICC_{50}$ von VDV1 oder VDV2 enthält und das tetravalente Vakzin $10^5$ $DICC_{50}$ von CYD DEN-1, 2, 3 und $10^3$ $DICC_{50}$ von CYD DEN-4 enthält.

**13.** Impfviren des Dengue-Fiebers zur Verwendung bei einer Immunisierung gegen das Virus des Dengue-Fiebers, das Folgendes umfasst:

(a) eine erste Verabreichung eines monovalenten Vakzins, das ein Impfvirus des Dengue-Fiebers eines ersten Serotyps enthält, in dem das Impfvirus ausgewählt ist aus der Gruppe bestehend aus Stämmen VDV1, deren komplette Sequenz in der SEQ ID NR:1 angegeben ist, und VDV2, deren komplette Sequenz in der SEQ ID NR:2 angegeben ist,
(b) eine zweite Verabreichung eines tetravalenten Vakzins, das Impfviren der 4 Serotypen 1 bis 4 des Dengue-Fiebers enthält und bei dem das tetravalente Vakzin wenigstens 30 Tage bis höchstens 12 Monate nach der Verabreichung des monovalenten Vakzins verabreicht wird.

**14.** Monovalentes Vakzin, das ein Impfvirus des Dengue-Fiebers eines ersten Serotyps enthält, bei dem das Impfvirus ausgewählt ist aus der Gruppe bestehend aus Stämmen VDV1, deren komplette Sequenz in der SEQ ID NR:1 angegeben ist, und VDV2, deren komplette Sequenz in der SEQ ID NR:2 angegeben ist, zur Verwendung in einem Verfahren, das die Einleitung einer Antikörperreaktion zulässt, die gegen die 4 Serotypen des Dengue-Fiebers bei einem Patienten neutralisiert, das Folgendes beinhaltet:

(a) eine erste Verabreichung des monovalenten Vakzins;
(b) eine zweite Verabreichung eines tetravalenten Vakzins, das Impfviren von 4 Serotypen 1 bis 4 des Dengue-Fiebers umfasst und bei dem das tetravalente Vakzin wenigstens 30 Tage bis höchstens 12 Monate nach der Verabreichung des monovalenten Vakzins verabreicht wird.

**15.** Ein tetravalentes Vakzin, das Impfviren von 4 Serotypen 1 bis 4 des Dengue-Fiebers umfasst, zur Verwendung in einem Verfahren, das das Einleiten einer Antikörperreaktion zulässt, die gegen die 4 Serotypen des Dengue-Fiebers bei einem Patienten neutralisiert, das Folgendes beinhaltet:

(a) eine erste Verabreichung eines monovalenten Vakzins, das ein Virus des Dengue-Fiebers des ersten Serotyps umfasst, bei dem das genannte Impfvirus ausgewählt ist aus der Gruppe bestehend aus Stämmen VDV1, deren komplette Sequenz in der SEQ ID NR:1 angegeben ist, und VDV2, deren komplette Sequenz in der SEQ ID NR:2 angegeben ist;
(b) eine zweite Verabreichung des tetravalenten Vakzins, bei der das tetravalente Vakzin wenigstens 30 Tage bis höchstens 12 Monate nach der Verabreichung des monovalenten Vakzins verabreicht wird.

## Claims

**1.** Use of vaccinal viruses for dengue fever for the fabrication of a monovalent vaccine and a tetravalent vaccine for immunization against the dengue virus comprising:

(a) a first administration of a monovalent vaccine comprising a vaccinal virus for a first serotype of dengue fever wherein said vaccinal virus is selected from the group comprising the VDV1 strain the complete sequence of which is given in SEQ ID NO:1 and the VDV2 strain the complete sequence of which is given in SEQ ID NO:2,
(b) a second administration of a tetravalent vaccine comprising vaccinal viruses for the four serotypes 1 to 4 of dengue fever and wherein the tetravalent vaccine is administered at least 30 days and not more than 12 months after the administration of the monovalent vaccine.

**2.** Use of a vaccinal virus for a first serotype of dengue fever wherein said vaccinal virus is selected from the group comprising the VDV1 strain the complete sequence of which is given in SEQ ID NO:1 and the VDV2 strain the complete sequence of which is given in SEQ ID NO:2, for the fabrication of a monovalent vaccine for use in a method enabling induction of a neutralizing antibody response against the four serotypes of dengue fever in a patient, comprising:

(a) a first administration of the monovalent vaccine,
(b) a second administration of a tetravalent vaccine comprising vaccinal viruses for the four serotypes 1 to 4 of dengue fever and wherein the tetravalent vaccine is administered at least 30 days and not more than 12 months after the administration of the monovalent vaccine.

**3.** Use of vaccinal viruses for the four serotypes 1 to 4 of dengue fever for the fabrication of a tetravalent vaccine for use in a method enabling induction of a neutralizing antibody response against the four serotypes of dengue fever in a patient, comprising:

(a) a first administration of a monovalent vaccine comprising a vaccinal virus for a first serotype of dengue fever wherein said vaccinal virus is selected from the group comprising the VDV1 strain the complete sequence of which is given in SEQ ID NO:1 and the VDV2 strain the complete sequence of which is given in SEQ ID NO:2,
(b) a second administration of the tetravalent vaccine and wherein the tetravalent vaccine is administered at least 30 days and not more than 12 months after the administration of the monovalent vaccine.

**4.** Use according to any one of claims 1 to 3 wherein the vaccinal viruses used in the tetravalent vaccine are CYD DEN-1,2,3 and 4.

**5.** Use according to any one of claims 1 to 4 wherein the quantity of dengue fever vaccinal viruses of serotypes 1,2,3 and 4 lies within a range from $10^3$ to $10^6$ $DICC_{50}$.

6. Use according to any one of claims 1 to 5, wherein the monovalent vaccine comprises $10^4$ $DICC_{50}$ of VDV1 or VDV2 and the tetravalent vaccine comprises $10^5$ $DICC_{50}$ of CYD DEN-1,2,3 and 4.

7. Use according to any one of claims 1 to 5, wherein the monovalent vaccine comprises $10^4$ $DICC_{50}$ of VDV1 or VDV2 and the tetravalent vaccine comprises $10^5$ $DICC_{50}$ of CYD DEN-1,2,3 and $10^3$ $DICC_{50}$ of CYD DEN-4.

8. Use according to any one of claims 1 to 7, wherein the second administration (b) is performed 30 to 60 days after the first administration (a).

9. Immunization kit against the dengue fever virus comprising a box containing at least:

(a) a first container containing a monovalent vaccine comprising a vaccinal virus selected from the group comprising the VDV1 strain the complete sequence of which is given in SEQ ID NO:1 and the VDV2 strain the complete sequence of which is given in SEQ ID NO:2,
(b) a second container containing a tetravalent vaccine comprising vaccinal viruses for the four serotypes 1 to 4 of dengue fever.

10. Immunization kit according to claim 9 wherein the second container contains a tetravalent vaccine comprising the four CYD DEN-1, 2, 3 and 4.

11. Immunization kit according to claim 10 wherein the monovalent vaccine comprises $10^4$ $DICC_{50}$ of VDV1 or VDV2 and the tetravalent vaccine comprises $10^5$ $DICC_{50}$ of CYD DEN-1, 2, 3 and 4.

12. Immunization kit according to claim 10 wherein the monovalent vaccine comprises $10^4$ $DICC_{50}$ of VDV1 or VDV2 and the tetravalent vaccine comprises $10^5$ $DICC_{50}$ of CYD DEN-1, 2, 3 and $10^3$ $DICC_{50}$ of CYD DEN-4.

13. Vaccinal viruses for dengue fever for use in immunization against the dengue fever virus comprising:

(a) a first administration of a monovalent vaccine comprising a vaccinal virus for a first serotype of dengue fever wherein said vaccinal virus is selected from the group comprising the VDV1 strain the complete sequence of which is given in SEQ ID NO:1 and the VDV2 strain the complete sequence of which is given in SEQ ID NO:2,
(b) a second administration of a tetravalent vaccine comprising vaccinal viruses for the four serotypes 1 to 4 of dengue fever, and wherein the tetravalent vaccine is administered at least 30 days and not more than 12 months after the administration of the monovalent vaccine.

14. A monovalent vaccine comprising a vaccinal virus for a first serotype of dengue fever wherein said vaccinal virus is selected from the group comprising the VDV1 strain the complete sequence of which is given in SEQ ID NO:1 and the VDV2 strain the complete sequence of which is given in SEQ ID NO:2 for use in a method enabling induction of a neutralizing antibody response against the four serotypes of dengue fever in a patient, comprising:

(a) a first administration of the monovalent vaccine,
(b) a second administration of a tetravalent vaccine comprising vaccinal viruses for the four serotypes 1 to 4 of dengue fever and wherein the tetravalent vaccine is administered at least 30 days and not more than 12 months after the administration of the monovalent vaccine.

15. A tetravalent vaccine comprising vaccinal viruses for the four serotypes 1 to 4 of dengue fever for use in a method enabling induction of a neutralizing antibody response against the four serotypes of dengue fever in a patient, comprising:

(a) a first administration of a monovalent vaccine comprising a virus for a first serotype of dengue fever wherein said vaccinal virus is selected from the group comprising the VDV1 strain the complete sequence of which is given in SEQ ID NO:1 and the VDV2 strain the complete sequence of which is given in SEQ ID NO:2,
(b) a second administration of the tetravalent vaccine and

wherein the tetravalent vaccine is administered at least 30 days and not more than 12 months after the administration of the monovalent vaccine.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0266621 A
- WO 0057904 A
- WO 0057908 A
- WO 0057909 A
- WO 0057910 A
- WO 020950075 A
- WO 02102828 A
- WO 9837911 A
- WO 9640933 A
- WO 0160847 A
- EP 1159968 A
- WO 2006134433 A
- WO 03101397 A


### Littérature non-brevet citée dans la description


- **GÜBLER et al.** Epidemiology of arthropod-bome viral disease. CRC Press, 1988, 223-60
- **KAUTNER et al.** *J. of Pediatrics,* 1997, vol. 131, 516-524
- **RIGAU-PÉREZ et al.** *Lancet,* 1998, vol. 352, 971-977
- **VAUGHN et al.** *J Infect Dis,* 1997, vol. 176, 322-30
- **VAUGHN et al.** *J. Infect. Dis.,* 1997, vol. 176, 322-30
- WHO technical Guide. Dengue haemorrhagic fever: diagnosis, treatment and control. World Health Organization, 1986, 1-2
- **GUBLER.** *TRENDS in Microbiology,* 2002, vol. 10, 100-103
- **MONATH.** *Proc. Natl. Acad. Sci.,* 1994, vol. 91, 2395-2400
- **SHIRTCLIFFE et al.** *J. Roy. Coll. Phys. Lond.,* 1998, vol. 32, 235-237
- **DEFRAITES et al.** *MMWR 1994,* 1994, vol. 43, 845-848
- **WU et al.** *Nature Med.,* 2000, vol. 7, 816-820
- **SABIN.** *Am. J. Trop. Med. Hyg.,* 1952, vol. 1, 30-50
- **VAUGHN et al.** *J. Inf. Dis.,* 2000, vol. 181, 2-9
- **ROTHMAN ; ENNIS.** *Virology,* 1999, vol. 257, 1-6
- **THEILER M ; SMITH HH.** *J Exp. Med,* 1937, vol. 65, 767-786
- **RICE et al.** *Science,* 1985, vol. 229, 726-733
- **GALLER et al.** *Vaccines,* 1998, vol. 16 (9/10), 1024-1028